(19) Europäisches Patentamt
European Patent Office
Office européen des brevets

(11) EP 4 444 733 B1

(12) EUROPEAN PATENT SPECIFICATION

(45) Date of publication and mention of the grant of the patent:
**01.07.2026 Bulletin 2026/27**

(21) Application number: **22941858.7**

(22) Date of filing: **09.12.2022**

(51) International Patent Classification (IPC):
***C07G 1/00*** *(2011.01)* ***C08J 11/16*** *(2006.01)*
***B01J 3/04*** *(2006.01)* ***B01J 19/24*** *(2006.01)*
***C07C 45/32*** *(2006.01)*

(52) Cooperative Patent Classification (CPC):
(C-Sets available)
**C07C 45/32; C07G 1/00; C08J 11/16;** B01J 3/04; B01J 19/243; B01J 19/2475; B01J 2219/00157; C08J 2397/00 (Cont.)

(86) International application number:
**PCT/US2022/052348**

(87) International publication number:
**WO 2023/244264 (21.12.2023 Gazette 2023/51)**

(54) **FLOW-BASED AEROBIC DEPOLYMERIZATION OF LIGNIN**

AEROBE DEPOLYMERISIERUNG VON LIGNIN AUF STRÖMUNGSBASIS

DÉPOLYMÉRISATION AÉROBIE À BASE DE FLUX DE LIGNINE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **09.12.2021 US 202163287592 P**

(43) Date of publication of application:
**16.10.2024 Bulletin 2024/42**

(73) Proprietor: **Wisconsin Alumni Research Foundation
Madison, WI 53726 (US)**

(72) Inventors:
- **STAHL, Shannon
Madison, WI 53711 (US)**
- **WEEDA, Eric
Madison, WI 53705 (US)**
- **HOLLAND, Christopher
Madison, WI 53703 (US)**
- **ROOT, Thatcher
Madison, WI 53711 (US)**

(74) Representative: **Predazzi, Valentina et al
Società Italiana Brevetti S.p.A.
Piazza di Pietra, 39
00186 Roma (IT)**

(56) References cited:
**WO-A1-2014/068590 WO-A1-2020/181171**

- **ARAUJO J D P ET AL: "Structured packed bubble column reactor for continuous production of vanillin from Kraft lignin oxidation", CATALYSIS TODAY, ELSEVIER, AMSTERDAM, NL, vol. 147, 1 September 2009 (2009-09-01), pages S330 - S335, XP026545884, ISSN: 0920-5861, [retrieved on 20090730], DOI: 10.1016/J.CATTOD.2009.07.016**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C07C 45/32, C07C 47/575;**
**C07C 45/32, C07C 47/58**

## Description

CROSS-REFERENCE TO RELATED APPLICATIONS

Priority is claimed to US provisional application 63/287,592, filed December 9, 2021.

STATEMENT REGARDING FEDERALLY SPONSORED RESEARCH

This invention was made with government support under DE-SC0018409 and DE-EE0008148 awarded by the US Department of Energy. The government has certain rights in the invention.

FIELD OF THE INVENTION

The invention is directed to the aerobic depolymerization of lignin.

BACKGROUND

The valorization of lignin to valuable chemicals is a key area in moving to renewable chemical feedstocks. One method to valorize lignin to vanillin and other small aromatic monomers is an aerobic copper catalyzed alkaline reaction typically performed in batch reactors with temperatures exceeding 160 °C and reaction times from 10 to 45 minutes (Bjørsvik et al. 1999). The amount of vanillin and other aromatic monomers is limited by a competition between monomer production and monomer degradation. Scale up of current batch processes are further limited due to slow heat up times and poor liquid-oxygen mixing during the reaction. To avoid the slow heat up times, some systems employ flow reactors capable of rapidly heating and cooling lignin solutions. To address the poor liquid-oxygen mixing, some systems employ a lignin stream saturated with oxygen (Roberts et al. 2011). Unfortunately, the oxygen could be easily consumed during the reaction. Other systems employ a structured bubble column to increase oxygen and lignin mixing (Aráujo et al. 2009). However, the lack of independent control of the oxygen throughout the length of the reactor leads to rapid consumption of oxygen and rapid monomer degradation.

Methods and systems of depolymerizing lignin that address at least some of the deficiencies outlined above are needed.

SUMMARY OF THE INVENTION

The invention is directed to methods and systems of depolymerizing lignin. The methods and systems generate aromatic monomers from lignin faster and at higher yields than conventional methods.

The methods comprise flowing an aqueous reaction medium comprising the lignin and a base through a lumen of a channel at a temperature and for a time effective to depolymerize at least a portion of the lignin to aromatic monomers. The channel preferably comprises an oxygen-permeable channel substrate comprising an inner surface facing the lumen and an outer surface facing away from the lumen, wherein the outer surface of the oxygen-permeable channel substrate is in contact with oxygen gas. Some versions comprise flowing the reaction medium through the lumen of the channel in the presence of an oxidation catalyst. Some versions comprise flowing an aqueous reaction medium comprising the lignin, the base, and an oxidation catalyst through the lumen of the channel at a temperature and for a time effective to depolymerize at least a portion of the lignin to aromatic monomers.

In exemplary versions, the channel is fluoropolymer membrane tubing (e.g., polytetrafluoroethylene, fluorinated ethylene propylene, perfluoroalkoxy alkane, and 2,2-bistrifluoromethyl-4,5-difluoro-1,3-dioxole/tetrafluoroethylene copolymer) running through an oxygen-pressurized tube-in-shell reactor that is heated to a reaction temperature ranging from about 200 °C to about 250° C with a furnace; the base is a strong base such as NaOH; the oxidation catalyst is a copper salt such as $CuSO_4$; and the flowing is performed for a time within a range from 5 seconds to 500 seconds. The methods and systems are capable of generating such aromatic monomers as vanillin, syringaldehyde, p-hydroxybenzoic acid, vanillic acid, syringic acid, acetovanillone, and acetosyringone in a total amount of at least 20% w/w of the lignin originally present in the reaction medium. An exemplary system and aspects of an exemplary method are shown in FIG. 1.

The objects and advantages of the invention will appear more fully from the following detailed description of the preferred embodiment of the invention made in conjunction with the accompanying drawings.

BRIEF DESCRIPTION OF THE DRAWINGS

FIG. 1. An exemplary membrane reactor (a) and reaction (b) of the present invention.
FIG. 2. Comparison of alkaline oxidation methods of depolymerizing lignin into aromatic compounds. a) General schema of alkaline oxidation. b) Batch method of the prior art and characteristics thereof. c) Flow oxidation of lignin in accordance with the present invention using a permeable membrane flow reactor system and characteristics thereof, including minimized aromatic monomer degradation.
FIG. 2: Exemplary Reaction time course of flow oxidation in accordance with the present invention using different tubing with different surface area to volume ratios
FIG. 3: Time course of exemplary alkaline oxidation of lignin in accordance with the present invention using different tubing materials (Teflon™ PFA and PTFE).
FIG. 4: Time course of exemplary alkaline oxidation of lignin in accordance with the present invention using different inner diameters of tubing.
FIG. 5: Time course of exemplary alkaline oxidation of lignin in accordance with the present invention using different loading of lignin.
FIG. 6: Time course of exemplary alkaline oxidation of lignin in accordance with the present invention using different pressures of oxygen or nitrogen.
FIG. 7: Time course of exemplary alkaline oxidation of lignin in accordance with the present invention using different temperatures.
FIG. 8: Time course of exemplary alkaline oxidation of lignin in accordance with the present invention using different loading of copper.
FIGS. 9A and 9B. Depolymerization of lignin from a variety of different tree sources prepared using a variety of different isolation methods. FIG. 9A. Reactivity of CuAHP poplar lignin, GVL-extracted poplar lignin, glyoxylic acid-stabilized birch lignin, CuAHP pine lignin, glyoxylic acid-stabilized pine lignin, and Kraft pine lignin using CuO oxidations (Conditions A), $CuSO_4$ batch oxidations (Conditions B), and CuSO4 flow oxidations (Conditions C). The different shading of the bars for the CuAHP poplar lignin (Poplar CuAHP) and GVL-extracted poplar lignin (Poplar GVL) indicate relative yields of, in order from bottom to top: vanillic acid, syringic acid, vanillin, syringaldehyde, acetovanillone, acetosyringone, and p-hydroxybenzoic acid. The different shading of the bars for the glyoxylic acid-stabilized birch lignin (Birch Glyox stabilized) indicate relative yields of, in order from bottom to top: vanillic acid, syringic acid, vanillin, syringaldehyde, acetovanillone, and acetosyringone. The different shading of the bars for the CuAHP pine lignin (Pine CuAHP), glyoxylic acid-stabilized pine lignin (Pine Glyox stabilized), and Kraft pine lignin (Pine Kraft) indicate relative yields of, in order from bottom to top: vanillic acid, vanillin, and acetovanillone. FIG. 9B. depolymerization of various lignins using Conditions C: "CuAHP" is CuAHP poplar lignin. "Glyox stabilized" is glyoxylic acid-stabilized birch lignin. "GVL" is GVL-extracted poplar lignin. "MA hydrotropic" is maleic acid hydrotropic-extracted lignin from birch. "CuAHP Pine" is CuAHP pine lignin. "Kraft" is Kraft pine lignin. "Low Sulfur Kraft" is Lignin obtained from a modified Kraft pulping method using low amounts of sulfides. "LS-Ca Salt" is lignosulfonate calcium salt softwood lignin. "Glyox Pine" is glyoxylic acid stabilized pine lignin.
FIGS. 10A and 10B. Simple kinetic modelling of lignin depolymerization of the invention using experimentally determined diffusion and reaction constants. Modeling of the reaction shows that the reactivity of the lignin is improved by metering in the oxygen using the permeable membrane, allowing for high yields.
FIGS. 11A-11V. Exemplary phenolic and benzoquinone monomers shown or predicted to be produced with the methods of the invention.

DETAILED DESCRIPTION OF THE INVENTION

The term "channel" refers to a conduit capable of flowing liquid reaction medium therethrough. The channel preferably has at least one input, at least one output, and an enclosed lumen running the length between the at least one input and at least one output. The channel can be unbranched or branched, can have one or multiple inputs, and can have one or multiple outputs.

The channel comprises a channel substrate that defines the lumen of the channel. The channel substrate can be composed of a single material or multiple different materials. The channel substrate comprises an inner surface that faces - and defines - the lumen through which the reaction medium flows.

At least a portion of the channel substrate comprises oxygen-permeable channel substrate that is composed of a material substantially permeable to liquid but permeable to oxygen. The oxygen-permeable channel substrate is preferably disposed along a given length of the channel along the direction of flow. In some versions, the oxygen-permeable channel substrate is disposed along the entire length of the channel along the direction of flow. In some versions, the oxygen-permeable channel substrate spans the entire circumference (for channels having a curvilinear (e.g., circular) cross section) or all sides (for channels having a polygonal (e.g., triangular, square, rectangular) cross section) of the channel. In some versions, the oxygen-permeable channel substrate spans only a portion of the circumference (for channels having a curvilinear (e.g., circular) cross section) or fewer than all sides (for channels having a polygonal (e.g.,

triangular, square, rectangular) cross section) of the channel.

The channel can take any of number of forms. In some versions, the channel takes the form of tubing. The tubing can be entirely or partially composed of the oxygen-permeable channel substrate, such as oxygen-permeable membrane that is formed in tubular form. In some versions, the channel takes the form of etching in a substantially solid, nonpermeable substrate (e.g., a solid heating block) that is covered and sealed with an oxygen-permeable membrane or other oxygen-permeable channel substrate. Other forms are encompassed by the present invention.

The oxygen-permeable channel substrate, as outlined above, can comprise a membrane. The membrane can be synthetic or non-synthetic. The membrane can be a polymeric membrane. "Polymeric membrane" refers to a membrane composed of one or more polymers. The polymeric membrane can be a fluoropolymer membrane. "Fluoropolymer membrane" refers to a membrane comprising a fluoropolymer.

Fluoropolymers are fluorocarbon-based polymers with multiple carbon-fluorine bonds. Exemplary fluoropolymers include polytetrafluoroethylene (PTFE), fluorinated ethylene propylene (FEP), perfluoroalkoxy alkane (PFA), and 2,2-bistrifluoromethyl-4,5-difluoro-1,3-dioxole/tetrafluoroethylene copolymer (AF). PTFE, FEP, PA, and AF are sold under the brand names Teflon™, Teflon™ FEP, Teflon™ PFA (perfluoroalkoxy alkane), and Teflon™ AF-1600 or Teflon™ AF-2400, respectively, by the Chemours Company FC, LLC (Wilmington, DE). FEP is a copolymer of hexafluoropropylene and tetrafluoroethylene. PFA is also known as perfluoroalkoxy polymer resins, perfluoroalkoyl alkanes, and perfluoroalkoyl polymer resins. PFA is a copolymer of tetrafluoroethylene ($C_2F_4$) and perfluoroethers ($C_2F_3OR^f$, where $R^f$ is a perfluorinated group such as trifluoromethyl ($CF_3$)) (Giocobbe 1990). AF is an amorphous, perfluorinated copolymer containing 2,2-bistrifluoromethyl-4,5-difluoro-1,3-dioxole and tetrafluoroethylene (Pinnau 1996). Other exemplary fluoropolymers include polyvinylfluoride (PVF), polyvinylidene fluoride (PVDF), polychlorotrifluoroethylene (PCTFE), polyethylenetetrafluoroethylene (ETFE), polyethylenechlorotrifluoroethylene (ECTFE), perfluorinated elastomer (perfluoroelastomer) (FFPM/FFKM), vinylidene fluoride-based copolymer fluoroelastomers (FPM/FKM), tetrafluoroethylene-propylene-based fluoroelastomers (FEPM), perfluoropolyether (PFPE), perfluorosulfonic acid (PFPE), and perfluoropolyoxetane.

The polymeric membrane can comprise other polymers, such as cellulose acetate, nitrocellulose, cellulose ester, polysulfone (PS), polyether sulfone (PES), polyacrilonitrile (PAN), polyamide, polyimide, polyethylene (PE), polypropylene (PP), and polyvinylchloride (PVC), among others.

"Aqueous reaction medium" refers to a reaction medium containing water in an amount of at least 70% v/v, such as at least 75% v/v, at least 80% v/v, at least 85% v/v, at least 90% v/v, at least 95% v/v, or at least 99% v/v.

The lignin included in the reaction medium can comprise any form of lignin. Lignin is a structurally complex heterogeneous aromatic biopolymer made of three principal building blocks, p-hydroxyphenol (H), guaiacyl (G), and syringyl (S) units in various combinations and amounts. The lignin in the reaction medium can include native lignin or processed lignin. "Native lignin" refers to lignin that has not been processed from its native, unaltered chemical state by any pretreatments except for mechanical comminution (chopping, chipping, grinding, milling, *etc.*). "Processed lignin" is lignin that has been chemically altered from its native state by one or more pretreatments other than mechanical comminution. "Pretreatment" used in the context of treating native lignin is a term well understood in the art that encompasses a number of treatments that change the physical and/or chemical structure of lignocellulosic biomass for downstream treatments, such as lignin depolymerization, cellulose hydrolysis, or other treatments. For descriptions of various pretreatments, see Kumar et al. 2017 and Kumar et al. 2009.

Types of pretreatments include physical pretreatments, chemical pretreatments, physicochemical pretreatments, and biological pretreatments. Physical pretreatments include mechanical comminution *(e.g.,* chipping, grinding, milling), microwave irradiation, ultrasound sonication, pyrolysis, and pulsed-electric field treatment. Chemical pretreatments include acid treatment *(e.g.,* sulfuric acid treatment, dicarboxylic acid treatment) alkali treatment, ozonolysis, organosolv treatment, ionic liquid treatment, deep eutectic solvent treatment, and natural deep eutectic solvent treatment. Physicochemical pretreatments include steam explosion, liquid hot water treatment, wet oxidation, SPORL (sulfite pretreatment to overcome recalcitrance of lignocellulose) pretreatment, ammonia-based pretreatment (*e.g.*, ammonia fiber explosion (AFEX), ammonia recycle percolation (ARP), soaking aqueous ammonia (SAA), $CO_2$ explosion, and oxidative pretreatment. Biological pretreatments include fungi treatment *(e.g.,* brown-rot fungi treatment, white-rot fungi treatment, soft-rot fungi treatment), bacterial treatment, archaeal treatment, and enzyme treatment *(e.g.,* peroxidase enzyme treatment, laccase enzyme treatment). Merely washing lignocellulosic biomass with non-chemically reactive solvents such as organic solvents *(e.g.,* dioxane) or water at a temperature under about 100°C and a pressure at or near atmospheric does not constitute a lignocellulosic pretreatment. Organosolv methods, for example, typically employ temperatures above 140°C and elevated pressures.

In some versions of the invention, the lignin included in the reaction medium comprises lignin previously treated with physical pretreatment. In some versions of the invention, the lignin included in the reaction medium comprises lignin previously treated with chemical pretreatment. In some versions of the invention, the lignin included in the reaction medium comprises lignin previously treated with physicochemical pretreatment. In some versions of the invention, the lignin included in the reaction medium comprises lignin previously treated with biological pretreatment. In some versions of the

invention, the lignin included in the reaction medium comprises lignin previously treated with mechanical comminution *(e.g.,* chipping, grinding, milling). In some versions of the invention, the lignin included in the reaction medium comprises lignin previously treated with microwave irradiation. In some versions of the invention, the lignin included in the reaction medium comprises lignin previously treated with ultrasound sonication. In some versions of the invention, the lignin included in the reaction medium comprises lignin previously treated with pyrolysis. In some versions of the invention, the lignin included in the reaction medium comprises lignin previously treated with pulsed-electric field treatment. In some versions of the invention, the lignin included in the reaction medium comprises lignin previously treated with acid treatment *(e.g.,* sulfuric acid treatment, dicarboxylic acid treatment). In some versions of the invention, the lignin included in the reaction medium comprises lignin previously treated with alkali treatment. In some versions of the invention, the lignin included in the reaction medium comprises lignin previously treated with ozonolysis. In some versions of the invention, the lignin included in the reaction medium comprises lignin previously treated with organosolv treatment. In some versions of the invention, the lignin included in the reaction medium comprises lignin previously treated with ionic liquid treatment. In some versions of the invention, the lignin included in the reaction medium comprises lignin previously treated with deep eutectic solvent treatment. In some versions of the invention, the lignin included in the reaction medium comprises lignin previously treated with natural deep eutectic solvent treatment. In some versions of the invention, the lignin included in the reaction medium comprises lignin previously treated with steam explosion. In some versions of the invention, the lignin included in the reaction medium comprises lignin previously treated with liquid hot water treatment. In some versions of the invention, the lignin included in the reaction medium comprises lignin previously treated with wet oxidation. In some versions of the invention, the lignin included in the reaction medium comprises lignin previously treated with SPORL (sulfite pretreatment to overcome recalcitrance of lignocellulose) pretreatment. In some versions of the invention, the lignin included in the reaction medium comprises lignin previously treated with ammonia-based pretreatment. In some versions of the invention, the lignin included in the reaction medium comprises lignin previously treated with ammonia fiber explosion (AFEX). In some versions of the invention, the lignin included in the reaction medium comprises lignin previously treated with ammonia recycle percolation (ARP). In some versions of the invention, the lignin included in the reaction medium comprises lignin previously treated with soaking aqueous ammonia (SAA) treatment. In some versions of the invention, the lignin included in the reaction medium comprises lignin previously treated with $CO_2$ explosion. In some versions of the invention, the lignin included in the reaction medium comprises lignin previously treated with mild acidolysis treatment. In some versions of the invention, the lignin included in the reaction medium comprises lignin previously treated with oxidative pretreatment. In some versions of the invention, the lignin included in the reaction medium comprises lignin previously treated with fungi treatment. In some versions of the invention, the lignin included in the reaction medium comprises lignin previously treated with brown-rot fungi treatment. In some versions of the invention, the lignin included in the reaction medium comprises lignin previously treated with white-rot fungi treatment. In some versions of the invention, the lignin included in the reaction medium comprises lignin previously treated with soft-rot fungi treatment. In some versions of the invention, the lignin included in the reaction medium comprises lignin previously treated with bacterial treatment. In some versions of the invention, the lignin included in the reaction medium comprises lignin previously treated with archaeal treatment. In some versions of the invention, the lignin included in the reaction medium comprises lignin previously treated with enzyme treatment. In some versions of the invention, the lignin included in the reaction medium comprises lignin previously treated with peroxidase enzyme treatment. In some versions of the invention, the lignin included in the reaction medium comprises lignin previously treated with laccase enzyme treatment. In some versions, the lignin included in the reaction medium comprises lignin pretreated with copper-catalyzed alkaline hydrogen peroxide (Cu-AHP) pretreatment ("CuAHP lignin") (see, *e.g.*, US 2020/0332376 A1 to Hegg et al.). In some versions, the lignin included in the reaction medium comprises lignin pretreated with aldehyde stabilizing groups and mild hydrochloric acid pretreatment ("Glyoxylic acid stabilized lignin") (see, *e.g.,* US10906856B2 to Bertella et al.). In some versions, the lignin included in the reaction medium comprises lignin pretreated with an oxidation of the benzyl alcohol or the primary alcohol of the lignin (see, *e.g.,* US8969534B2). In some versions of the invention, the lignin included in the reaction medium comprises Kraft lignin. In some versions of the invention, the lignin included in the reaction medium comprises sulfonated lignin (*e.g.*, sodium lignosulfonate lignin). In some versions, the lignin included in the reaction medium comprises lignin pretreated with γ-valerolactone (GVL) pretreatment ("γ-valerolactone lignin" or "GVL lignin") (see, e.g., Gelosia et al. 2017, Fang et al. 2015, Le and Ma et al. 2016, Le and Zaitseva et al. 2016, Luterbacher et al. 2014, Luterbacher et al. 2015, and Wu et al. 2016).

Other methods of depolymerizing lignin are known from WO2014/068590, WO2020/181171 and from Araujo J. D. P. in Catalysis Today 2009, Vol. 147, pages S330-S335.

The lignin can be derived from any source, such as corn cobs, corn stover, cotton seed hairs, grasses, hardwood stems, leaves, newspaper, nut shells, paper, softwood stems, switchgrass, waste papers from chemical pulps, wheat straw, wood, woody residues, and other sources.

The lignin can be included in the reaction medium in an amount up to about 50% w/w or more. In various versions, the lignin is included in the rection medium in an amount of about 0.001% w/w, about 0.005% w/w, about 0.01% w/w, about 0.05% w/w, about 0.1% w/w, about 0.5% w/w, about 1% w/w, about 5% w/w, about 10% w/w, about 15% w/w, about 20%

w/w, about 25% w/w, about 30% w/w, about 35% w/w, about 40% w/w, about 45% w/w, about 50% w/w, or a value within a range between any two of the foregoing values. Exemplary ranges include from about 0.05% w/w to about 5% w/w, such as about 0.1% w/w to about 1% w/w. In some versions, the lignin is included in the reaction medium in an amount of about 50% w/w or less, about 45% w/w or less, about 40% w/w or less, about 35% w/w or less, about 30% w/w or less, about 25% w/w or less, about 20% w/w or less, about 15% w/w or less, about 10% w/w or less, or about 5% w/w or less.

The base included in the reaction medium can be any base. In some versions, the base included in the reaction medium is a strong base. Exemplary strong bases include sodium hydroxide, lithium hydroxide, potassium hydroxide, rubidium hydroxide, cesium hydroxide, calcium hydroxide, strontium hydroxide, and barium hydroxide. The base can be included in the reaction medium in an amount from 0.1 M or less to 4 M or more. In various versions, the base is included in the reaction medium in an amount of about 0.01 M, about 0.05 M, about 0.1 M, to about 0.5 M, about 1 M, about 2 M, about 3 M, about 4 M, about 5 M or a value within a range between any two of the foregoing values. An exemplary range is from about 1 M to about 4 M.

The oxidation catalyst can include any catalyst capable of catalyzing an oxidation reaction. The oxidation catalyst can include a metal-based catalyst, a non-metal-based catalyst, a homogeneous catalyst, a heterogeneous catalyst, or any combination thereof. See Kaur et al. 2013 for examples of metal-based catalysts and non-metal-based catalysts. The catalyst may be present within the system in any number of configurations. In some versions, the oxidation catalyst can be included in the reaction medium flowed through channel. In some versions, the catalyst is immobilized on or to the channel, such as on or to the inner surface of the channel substrate. In some versions, the catalyst forms a part of the channel substrate itself.

In some versions, the oxidation catalyst comprises a metal-based catalyst. A metal-based catalyst is a catalyst that comprises a metal. The metal can be any metal described in any catalyst herein. The metal can comprise or consist of a non-noble metal. "Non-noble metal" is used herein to refer to a metal that is not a noble metal. "Noble metal" is used herein to refer to ruthenium (Ru), rhodium (Rh), palladium (Pd), silver (Ag), osmium (Os), iridium (Ir), platinum (Pt), and gold (Au). The metal can comprise or consist of a transition metal. The metal can comprise or consist of a first-row transition metal. "First-row transition metal" refers to transition metals in the first row of the periodic table, *i.e.* scandium (Sc), titanium (Ti), vanadium (V), chromium (Cr), manganese (Mn), iron (Fe), cobalt (Co), nickel (Ni), copper (Cu), and zinc (Zn). In some versions, the metal can comprise or consist of titanium (Ti), vanadium (V), chromium (Cr), manganese (Mn), iron (Fe), cobalt (Co), nickel (Ni), and/or copper (Cu). In some versions, the metal can comprise or consist of manganese (Mn), iron (Fe), cobalt (Co), nickel (Ni), vanadium (V), and/or copper (Cu).

In some versions, the metal-based catalyst is provided in the form of a metal salt. In some versions, the metal-based catalyst is provided in the form of a metal hydroxide. In some versions, the metal-based catalyst is provided in the form of a metal oxide. "Provided" in this context refers to the state of the catalyst when initially added to a reaction medium. It is understood that most metal salts will form hydroxides in solution with base. The metal in the salt, hydroxide, or oxide forms can be in any oxidation state. Exemplary oxidation states include +2, +3, or +4 oxidation states. Exemplary metal salts include manganese(II) chloride ($MnCl_2$), manganese(II) sulfate ($MnSO_4 \cdot H_2O$), iron(II) chloride ($FeCl_2$), iron(II) sulfate ($FeSO_4 \cdot$H2O), cobalt(II) chloride ($CoCl_2$), cobalt(II) sulfate ($CoSO_4 \cdot H_2O$), nickel(II) chloride ($NiCl_2$), nickel(II) sulfate ($NiSO_4 \cdot H_2O$), copper(II) chloride ($CuCl_2$), and copper(II) sulfate ($CuSO_4 \cdot H_2O$). Other oxidation states of the metals and other anions are acceptable. Exemplary metal hydroxides include manganese(II) hydroxide ($Mn(OH)_2$), iron(II) hydroxide ($Fe(OH)_2$), cobalt(II) hydroxide ($Co(OH)_2$), nickel(II) hydroxide ($Ni(OH)_2$), and copper(II) hydroxide ($Cu(OH)_2$). Other oxidation states of the metals are acceptable. Exemplary metal oxides include manganese(II) oxide (MnO), iron(II) oxide (FeO), cobalt(II) oxide (CoO), nickel(II) oxide (NiO), and copper(II) oxide (CuO). Other oxidation states of the metals are acceptable.

In some versions of the invention, the oxidation catalyst is a heterogeneous catalyst. Nonlimiting examples of heterogeneous oxidation catalysts include Co-PANI-C, Fe-PANI-C, FeCo-PANI-C, Co-Phen-C, $Co_3O_4$, $Fe_2O_3$, $Mn_2O_3$ or other manganese oxides, CuO, Pd/C, Pt/C, Ru/C, Ni/C, Fe nanocatalyst, other metal on nitrogen-doped carbon (M-N-C) catalysts containing Fe or Co (for example, such as those used for electrochemical dioxygen reduction, see Sun et al. 2017 or Gewirth et al. 2018), $Fe_2O_3$ on silica, $Al_2O_3$, $TiO_2$, or other metal-oxide support, $Co_3O_4$ on silica, $Al_2O_3$, $TiO_2$, or other metal oxide support, supported Fe, Co, Ni, Cu or Mn phthalocyanines or porphyrins, vanadium oxides, Fe, Co, Ni, Cu, or Mn in zeolites such as ZSM-5, $MnO_x$ on alumina, mixed Mn-Co oxides, Au/$Al_2O_3$, Au/C, Au/Pt bimetallic nanoparticles, gold nanoparticles supported on $Mg(OH)_2$ nano sheets, Au/$TiO_2$ supported on ferritic stainless steel monoliths, nanoporous gold, P123-stabilized Au-Ag alloy, alumina-supported gold-ruthenium bimetallic catalysts, Au/CuO catalysts, cerium modified silver, Pd-Au catalyst, Au/ZnO, Au/$TiO_2$, microstructured Au/Ni-fiber, nanocrystalline Ag and Au-Ag alloys supported on titania, nanosized Au supported on 3-D ordered mesoporous $MnO_2$, Au/$FeO_x$, nanosized ruthenium particles decorated carbon nanofibers, Au/C, CeAlPO-5 molecular sieves, nanosized gold on $SiO_2$, Au/$SiO_2$, nano gold-mesoporous silica, nanosized gold, Ag/SBA-15, bimetallic Au-Pd/MgO, inverse $Fe_2O_3$/Au(111) model catalysts, silica-supported Au-Cu alloy, gold nanoparticles supported on MgO, silica-supported Au-$CuO_x$, Au/$Al_2O_3$, Au-Pd/C, Pd-Te supported catalysts, gold nanoparticles supported on functionalized mesoporous silica, silica supported cobalt (II) salen complex, gold nanowires, $Cu_{3/2}[PMo_{12}O_{40}]/SiO_2$, gold nanoparticles deposited on cellulose, metalloporphyrin bound to

silica, hydrophobized palladium, supported gold catalysts, Au/HMS catalysts, mobilized gold nanoparticles, mesoporous $Co_3O_4$, mesoporous and Au/$Co_3O_4$, metal-organic framework supported gold nanoparticles, Pt/$Al_2O_3$, Au/$TiO_2$, Co(AcO)$_2$/Mn(AcO)$_2$, nickel substituted copper chromite spinels, gold catalysts, MCM-48 molecular sieve modified with $SnCl_2$, CuO-impregnated mesoporous silica, Au-CuO/$Al_2O_3$, Pt/$Al_2O_3$, manganese-containing mesoporous MCM-41 and Al-MCM-41 molecular sieves, Au/C, gold immobilized mesoporous silica, nitrous oxide over MFI zeolites, CoAPO-5 molecular sieves, Mn-containing MCM-41, Mn (Salen)/MCM-41, nanostructured $CuO_x$/$CeO_2$, nano-Au catalysts, heteropoly catalysts containing Ru(III) and Rh(III) particles, gold supported on ZnO and $TiO_2$, bismuth promoted palladium catalysts, or other metal oxides not named above. See Ali et al. 2014 for further details on the above-mentioned catalysts. As used herein, "PANI" is an abbreviation for polyaniline, and "Phen" is an abbreviation for 1,10-phenanthroline.

In some versions, the oxidation catalyst comprises a metal-containing nitrogen-doped carbon catalyst. Exemplary metal-containing nitrogen-doped carbon catalysts include metal-PANI/C or metal-Phen/C catalyst. Exemplary metals in the metal-containing nitrogen-doped carbon catalyst include the metals described above for the metal-based catalyst, such as Co and Fe, among others.

In some versions, the oxidation catalyst can comprise a non-metal-based catalyst. A non-metal-based catalyst is a catalyst that does not comprises a metal. Examples of non-metal-based catalysts include metal-free nitrogen-doped carbon.

The oxidation catalyst can comprise a solid support. The solid support can comprise any solid support used for any catalyst described herein. The solid support can comprise silica, carbon, clay, zeolite, nitrogen-containing carbon matrices, polymers (e.g., polyaniline polymers), metal oxides, metal nitrides, boron nitride, and other materials. The supports can be porous. The supports can be microporous (having an average pore diameter of less than 2 nm), mesoporous (having an average pore diameter between 2 nm and 50 nm), or macroporous (having an average pore diameter of greater than 50 nm).

In some versions, the oxidation catalyst is embedded in the channel. In some versions the oxidation catalyst is embedded by adsorption of the oxidation catalyst in or to the channel substrate, such as to the inner surface of the channel substrate.

The oxidation catalyst can be confined within a porous cage. The porous cage can be composed of steel alloys, titanium, or other non-reactive metals. In various versions, the porous cage has an average pore size of about 1 μm, about 10 μm, about 50 μm, about 100 μm, about 500 μm, about 1000 μm, about 2500 μm, about 5,000 μm, or any range between any two of the foregoing values. In preferred versions of the invention, the porous cage has an average pore size of from about 10 μm to about 70 μm, such as from about 20 μm to about 60 μm or from about 30 μm to about 50 μm.

In some versions, the depolymerization is performed in the absence of an oxidation catalyst. The depolymerization in such a case occurs through autoxidation by exposure oxygen.

The oxidation catalyst can be included in the reaction medium in an amount up to about 5 mM or more. In various versions, the oxidation catalyst is included in the reaction medium in an amount of about 0.001 mM, about 0.005 mM, about 0.01 mM, about 0.05 mM, about 0.1 mM, about 0.5 mM, about 1 mM, about 2 mM, about 3 mM, about 4 mM, about 5 mM, or a value within a range between any two of the foregoing values. Exemplary ranges include from about 0.01 mM to about 1 mM, or about 0.05 mM to about 0.5 mM. In some versions, the medium comprises the oxidation catalyst in an amount less than about 2 mM, less than about 1.5 mM, less than about 1 mM, or less than about 0.5 mM.

The reaction medium can be flowed through the channel at a temperature from about 150 °C or less to about 250 °C or more. In various versions, the flowing is conducted at a temperature of about 150 °C, about 155 °C, about 160 °C, about 165 °C, about 170 °C, about 175 °C, about 180 °C, about 185 °C, about 190 °C, about 195 °C, about 200 °C, about 205 °C, about 210 °C, about 215 °C, about 220 °C, about 225 °C, about 230 °C, about 235 °C, about 240 °C, about 245 °C, about 250 °C, or a temperature within a range between any two of the foregoing values. Exemplary ranges include from about 200 °C to about 250 °C, about 205 °C to about 250 °C, about 200 °C to about 230 °C, about 205 °C to about 230 °C, about 200 °C to about 225 °C, about 205 °C to about 225 °C, about 200 °C to about 220 °C, about 205 °C to about 220 °C, about 200 °C to about 215 °C, or about 205 °C to about 215 °C. The temperature can be applied by positioning the channel within a furnace or other heating source.

The oxygen gas in contact with the oxygen-permeable channel substrate is present at an oxygen partial pressure and therefore contacts the oxygen-permeable channel substrate at an oxygen partial pressure. The oxygen partial pressure can be about 10 psig (69 kPag) or less to about 200 psig (1380 kPag) or more. In various versions, the oxygen partial pressure is about 10 psig (69 kPag), about 11 psig (76 kPag), about 12 psig (83 kPag), about 13 psig (90 kPag), about 14 psig (97 kPag), about 15 psig (103 kPag), about 16 psig (110 kPag), about 17 psig (117 kPag), about 18 psig (124 kPag), about 19 psig (131 kPag), about 20 psig (138 kPag), about 25 psig (172 kPag), about 30 psig (207 kPag), about 35 psig (241 kPag), about 40 psig (276 kPag), about 45 psig (310 kPag), about 50 psig (345 kPag), about 55 psig (379 kPag), about 60 psig (414 kPag), about 65 psig (448 kPag), about 70 psig (483 kPag), about 75 psig (517 kPag), about 80 psig (552 kPag), about 85 psig (586 kPag), about 90 psig (621 kPag), about 95 psig (655 kPag), about 100 psig (689 kPag), about 105 psig (724 kPag), about 110 psig (758 kPag), about 115 psig (793 kPag), about 120 psig (827 kPag), about 125 psig (862 kPag), about 130 psig (896 kPag), about 135 psig (931 kPag), about 140 psig (965 kPag), about 145 psig (1000

kPag), about 150 psig (1034 kPag), about 155 psig (1069 kPag), about 160 psig (1103 kPag), about 165 psig (1138 kPag), about 170 psig (1172 kPag), about 175 psig (1207 kPag), about 180 psig (1241 kPag), about 185 psig (1276 kPag), about 190 psig (1310 kPag), about 195 psig (1345 kPag), about 200 psig (1379 kPag), or an oxygen partial pressure within a range between any two of the foregoing values. In various versions, the oxygen partial pressure is at least about 10 psig (69 kPag), about 15 psig (103 kPag), about 16 psig (110 kPag), about 17 psig (117 kPag), about 18 psig (124 kPag), about 19 psig (131 kPag), about 20 psig (138 kPag), or about 25 psig (172 kPag), and/or up to about 30 psig (207 kPag), about 35 psig (241 kPag), about 40 psig (276 kPag), about 45 psig (310 kPag), about 50 psig (345 kPag), about 55 psig (379 kPag), about 60 psig (414 kPag), about 65 psig (448 kPag), about 70 psig (483 kPag), about 75 psig (517 kPag), about 80 psig (552 kPag), about 85 psig (586 kPag), about 90 psig (621 kPag), about 95 psig (655 kPag), about 100 psig (689 kPag), about 105 psig (724 kPag), about 110 psig (758 kPag), about 115 psig (793 kPag), about 120 psig (827 kPag), about 125 psig (862 kPag), about 130 psig (896 kPag), about 135 psig (931 kPag), about 140 psig (965 kPag), about 145 psig (1000 kPag), about 150 psig (1034 kPag), about 155 psig (1069 kPag), about 160 psig (1103 kPag), about 165 psig (1138 kPag), about 170 psig (1172 kPag), about 175 psig (1207 kPag), about 180 psig (1241 kPag), about 185 psig (1276 kPag), about 190 psig (1310 kPag), about 195 psig (1345 kPag), or about 200 psig (1379 kPag), or more. The oxygen partial pressures contacting the oxygen-permeable channel substrate can be obtained by feeding the channel through a pressure vessel containing an appropriate concentration of oxygen gas.

The oxygen gas in contact with the oxygen-permeable channel substrate can be present in a mixture with other gases. Such other gases can include one or more inert gases. Examples of other gases include nitrogen; carbon dioxide; one or more noble gases such as helium (He), neon (Ne), argon (Ar), krypton (Kr), xenon (Xe), and radon (Rn); or any combination thereof. In some versions, the oxygen gas in contact with the oxygen-permeable channel substrate is provided in the form of air and is therefore present with other gases present in air (nitrogen, argon, carbon dioxide, and others). In some versions, the oxygen gas in contact with the oxygen-permeable channel substrate can be present in a mixture of gases in an amount of about 6% v/v or less to about 100% v/v. Exemplary amounts include about 6% v/v, about 10% v/v, about 15% v/v, about 20% v/v, about 25% v/v, about 30% v/v, about 35% v/v, about 40% v/v, about 45% v/v, about 50% v/v, about 55% v/v, about 60% v/v, about 65% v/v, about 70% v/v, about 75% v/v, about 80% v/v, about 85% v/v, about 90% v/v, about 95% v/v, about 100% v/v, or an amount within a range between any two of the foregoing values.

The reaction medium can be flowed for any time effective to depolymerize at least a portion of the lignin to aromatic monomers. In some versions, the reaction medium is flowed for a time from about 5 s or less to about 1000 s or more. In various versions, the reaction medium is flowed for a time of about 5 s, about 10 s, about 20 s, about 30 s, about 40 s, about 50 s, about 60 s, about 70 s, about 80 s, about 90 s, about 100 s, about 110 s, about 120 s, about 130 s, about 140 s, about 150 s, about 160 s, about 170 s, about 180 s, about 190 s, about 200 s, about 210 s, about 220 s, about 230 s, about 240 s, about 250 s, about 260 s, about 270 s, about 280 s, about 290 s, about 300 s, about 310 s, about 320 s, about 330 s, about 340 s, about 350 s, about 360 s, about 370 s, about 380 s, about 390 s, about 400 s, about 410 s, about 420 s, about 430 s, about 440 s, about 450 s, about 460 s, about 470 s, about 480 s, about 490 s, about 500 s, about 510 s, about 520 s, about 530 s, about 540 s, about 550 s, about 560 s, about 570 s, about 580 s, about 590 s, about 600 s, about 610 s, about 620 s, about 630 s, about 640 s, about 650 s, about 660 s, about 670 s, about 680 s, about 690 s, about 700 s, about 810 s, about 820 s, about 830 s, about 840 s, about 850 s, about 860 s, about 870 s, about 880 s, about 890 s, about 900 s, about 910 s, about 920 s, about 930 s, about 940 s, about 950 s, about 960 s, about 970 s, about 980 s, about 990 s, about 1000 s, or a time within a range between any two of the foregoing values. An exemplary range is from about 5 s to about 400 s. The reaction medium can be flowed at any rate to achieve an appropriate residence time within the channel under the specified conditions.

The aromatic monomers produced with the methods of the present invention can comprise phenolic and/or benzoquinone monomers. "Phenolic monomers" refers to compounds having one and only one phenolic group. The phenolic groups in the phenolic monomers can comprise *p*-hydroxyphenyl (H), guaiacyl (G), and/or syringyl (S) phenolic groups. The phenolic monomer products can comprise oxidized phenolic monomer products. The phenolic monomer products can have the following structure:

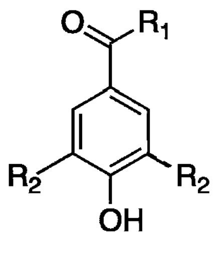

wherein $R_1$ is selected from the group consisting of H, OH, and $CH_3$, and each $R_2$ is independently selected from the group consisting of H and $OCH_3$. The phenolic monomer products can comprise phenolic monomer products comprising a

benzylic carbonyl. The term "benzylic" is used to describe the position of the first carbon bonded to a benzene, phenol, or other aromatic ring. Exemplary phenolic monomer products comprising a benzylic carbonyl include each of the compounds shown in FIGS. 11A-5V except for 2,6-dimethoxybenzoquinone. Exemplary aromatic monomers include vanillin, syringaldehyde, p-hydroxybenzoic acid, vanillic acid, syringic acid, acetovanillone, and acetosyringone, in addition to the other compounds shown in FIGS. 11A-5V.

The reaction medium can be flowed at least until the aromatic monomers reach a particular yield or concentration. In various versions, the reaction medium is flowed at least until the aromatic monomers (determined as total aromatic monomers) are produced in an amount of at least about 1% w/w, at least about 5% w/w, at least about 10% w/w, at least about 15% w/w, at least about 20% w/w, at least about 25% w/w, at least about 30% w/w, or at least about 35% w/w of the lignin originally present in the reaction medium.

As used herein, the singular forms "a," "an," and "the" include plural referents unless the content clearly dictates otherwise.

Numerical ranges as used herein are intended to include every number and subset of numbers contained within that range, whether specifically disclosed or not. Further, these numerical ranges should be construed as providing support for a claim directed to any number or subset of numbers in that range. For example, a disclosure of from 1 to 10 should be construed as supporting a range of from 2 to 8, from 3 to 7, from 5 to 6, from 1 to 9, from 3.6 to 4.6, from 3.5 to 9.9, and so forth.

It is understood that the invention is not confined to the particular construction and arrangement of parts herein illustrated and described, but embraces such modified forms thereof as come within the scope of the claims.

EXAMPLES

**Summary**

A permeable polytetrafluoroethylene (PTFE) membrane flow reactor was implemented in the alkaline aerobic depolymerization of lignin to increase monomer production and limit the subsequent destruction of lignin-derived aromatics by controlling the amount of oxygen (FIG. 1). The present flow-based methods offered control of parameters to enable monomer production and disfavor subsequent monomer degradation, such as precise reaction time control, rapid heating (*e.g.,* to temperatures >200 °C), and rapid cooling *(e.g.,* back to room temperature). This process was designed with an oxygen permeable PTFE membrane which allowed for a continual oxygen flux throughout the length of the reactor, abating concerns about rapid oxygen consumption as seen in other flow systems. The flow aerobic alkaline lignin depolymerization using a permeable membrane reactor has achieved aromatic monomer yields higher than 35 wt% using a source of CuAHP lignin (lignin pretreated with a copper-catalyzed alkaline hydrogen peroxide (Cu-AHP) pretreatment, as described in US 2020/0332376 A1 to Hegg et al.) (based on the weight of the originally lignin added). The majority of the aromatic monomers produced were vanillin, syringaldehyde, and p-hydroxybenzoic acid with small amounts of vanillic acid, syringic acid, acetovanillone, and acetosyringone. Results indicate scalability for a high yielding reaction for aerobic lignin depolymerization into valuable chemical feedstocks.

**Introduction**

Lignin is a heterogeneous biopolymer contained within the structure of lignocellulosic biomass in a wide variety of plant sources. Lignin is also the largest source of renewable aromatic compounds (FIG. 2, panel (a)). Valorization and upgrading of the lignin biopolymer are necessary steps to develop new chemical feedstocks and reduce reliance on fossil fuels (Tuck et al. 2012, Langholtz et al. 2016).

The heterogenous lignin biopolymer is comprised of many different types of units connected by both C-C and C-O bonds making full utilization or utilization of a large portion of the lignin a difficult challenge. In addition, different lignocellulosic biomass sources have different aromatic monomers. Hardwoods such as poplar and birch contain both S units (aromatics with two methoxy groups) as well as G units (aromatics with one methoxy group), while most softwoods only contain G units. Some biomass sources, such as poplar, have ester linkages, like p-hydroxybenzoate, attached to the lignin polymer.

Extraction and isolation of lignin has been an additional challenge to the valorization of lignin. Some common examples are the alkaline conditions used in technical lignin such as Kraft, as well as mildly alkaline oxidative conditions used in the CuAHP process and oxidative depolymerization. Lignin may also be isolated using acidic conditions such as the lignin extracted using the lignosulfonate process or in mild conditions using organic solvent such as the GVL process. However, extraction and isolation of the lignin from cellulose and hemicellulose has been difficult, as lignin may crosslink with itself in both the acidic and basic conditions commonly used to extract the lignin from the rest of the lignocellulosic biomass, making valorization and depolymerization of the lignin polymer more difficult. As such, Luterbacher and coworkers developed a protection method using aldehydes which limited lignin crosslinking allowing for high lignin

utilization. One such aldehyde is glyoxylic acid which creates a more aqueous soluble lignin.

Many methods to depolymerize lignin have been developed (FIG. 2, panel (b)). Common strategies include reductive methods, oxidative methods, and redox neutral methods. Oxidative methods commonly produce bifunctional aromatics which are particularly attractive for use as bio-based polymers (Llevot et al. 2016, Xanthopoulou et al. 2021, Bassett et al. 2020) and are promising feedstocks for microbial conversion due to the increased solubility in water (Beckham et al. 2016, Perez et al. 2019). Many methods have been developed with a two-stage oxidation followed by a depolymerization to achieve high yields of aromatic bifunctional monomers (Cui et al. 2021, Bosque et al. 2017, Lancefield et al. 2015, Rafiee et al. 2019, Das et al. 2018). However, many of these methods are not practical for large scale lignin depolymerizations due to the use of stoichiometric or otherwise expensive reagents. While there have been some recent developments in single-step oxidative catalytic fractionation methods, these methods are typically better suited for oxidation and depolymerization of lignin from whole lignocellulosic biomass (Luo et al. 2021, Du et al. 2021).

One strategy to process lignin into aromatics that has been prevalent for many years are variations of aqueous alkaline oxidative depolymerization methods. These methods have found wide usage in industrial conversion of waste lignin streams from pulp and paper industries to vanillin, such as the lignoboost method developed by Borregaard and Monsanto to produce vanillin from softwood lignosulfonates. Researchers have been able to isolate 5 of the aromatic products as pure compound. At one point, these alkaline aerobic methods were used to synthesize nearly 60% of the world vanillin supply (Hocking et al. 1997). While many variations of this reaction exist, typical conditions for alkaline oxidative lignin cleavage employ lignin loadings of 0.5-20 wt% in 2 M NaOH heated to 160-300 °C for anywhere from 45 minutes to 2 hours (Bjørsvik et al. 1999). Typical oxidants used are nitrobenzene, CuO, oxygen, or a combination of a copper salt and oxygen. Most methods that have been scaled to the hectogram scale use aerobic conditions.

Anaerobic methods (e.g. nitrobenzene, CuO) typically access higher yields of aromatics than aerobic methods. This has been attributed to a deleterious reaction with oxygen in the aerobic methods (Fargues et al. 1996). This aerobic reactivity with the aromatic monomers has been studied by Rodrigues and coworkers and is assumed to start with oxidation of the phenol followed by a ring opening reaction to generate small organic acids with many of the products unidentified. This reactivity has been shown to be accelerated by consumption of the base during the reactions, the concentration of the aromatics, and the oxygen pressure (Aráujo et al. 2009).

Some forms of stainless steel and Hastelloy steel have been shown to be incompatible with the use of strong base at high temperatures. In some cases, there has been signs of etching of the metal of the reactor over several cycles, and in one case there was an industrial explosion attributed to the use of base in a stainless steel reactor.

To avoid the degradation of the aromatic monomers during the heat up and cool down of a batch reactor, Tarabanko and coworkers designed a two-stage reactor that worked with whole biomass or with isolated lignins. The first stage used a continuous stirred-tank reactor (CSTR) to remove the lignin from the rest of the biomass or solubilize the lignin. The products of the first stage were then pumped into a second CSTR that had catalyst and high pressures of oxygen to convert the lignin to aromatics. This two-stage procedure was able to achieve yields like that achieved in batch, but the degradation reaction of the aromatics was not slowed down. Rodrigues and coworkers created a structured bubble column flow reactor (Srihar et al. 2005, Aráujo et al. 2009). This method similarly has not resulted in higher yields of aromatics than batch reactivity. Using a structured bubble column reactor has allowed for Rodrigues and coworkers to scale this up to nearly 40 L. Lercher and coworkers constructed a plug flow reactor designed to rapidly heat and cool the reaction. However, rather than cofeeding the oxygen with the reaction solvent, the solution was saturated with oxygen prior to the reaction. This unfortunately led to increased aerobic degradation of the aromatics due to the high pressures of oxygen needed to saturate the solvent. To combat this, Lercher and coworkers added boric acid to limit the autoxidation pathway which resulted in high yield of oil, but this still produced a host of different products (Roberts et al. 2011).

We show below an exemplary new method to depolymerize lignin. The method employs a tube-in-shell reactor with a permeable membrane, such as PTFE (which is compatible with base and high temperatures), to control oxygen flow in a continuous, aerobic lignin depolymerization reaction (FIG. 2, panel (c)).

## Material and Methods

### *General Considerations*

All reagents were used as received unless otherwise noted. NaOH pellets (Certified ACS grade), Methanol (HPLC grade), and Acetonitrile (HPLC grade) were purchased from Fischer Scientific. $CuSO_4 \cdot 5H_2O$ (ACS Reagent grade), $FeCl_3$, Cu ICP standards, 1,4-dimethoxybenzene, vanillin, vanillic acid, acetovanillone, syringaldehyde, syringic acid, and acetosyringone were purchased from Sigma-Aldrich. Air (breathing quality), nitrogen (industrial grade), and oxygen (industrial grade) cylinders were obtained from Airgas. All aqueous solutions were made with Type 1, ultra-pure water. The CuAHP 3030 Lignin sample was extracted by the following method from a sample of NM-6 sapwood *(Populus maximowiczii* × *P. nigra)* obtained from the Great Lakes Bioenergy Research Center in Madison, WI. The GVL extracted lignin was extracted by the following method from a sample of NM-6 sapwood *(Populus maximowiczii* × *P. nigra)* obtained

from the Great Lakes Bioenergy Research Center in Madison, WI.

The flow reactor was constructed from a 600 mL stainless steel Parr vessel, which had been modified by tapping two 1/4 inch (6 mm) NPT taps in the bottom of the vessel. The temperature was controlled by a Parr 4838 temperature controller with a K Type thermocouple. A Hitachi L6200 Intelligent pump was fitted with PTFE tubing purchased from Sigma-Aldrich (OD X ID X Length, 1/8" (3 mm) X 1.6 mm X 300" (760 cm), 1/16" (1.6 mm) X 0.8 mm X 300" (760 cm), 1/16" (1.6 mm) X 0.5 mm X 300" (760 cm), 1/16" (1.6 mm) X 0.3 mm X 300" (760 cm)) or Teflon™ PFA tubing (Chemours Company FC, LLC (Wilmington, DE)) purchased from McMaster Carr ( OD X ID X Length 1/8" (3 mm) X 1.6 mm X 300" (760 cm)). The tube pressure was controlled using a dome valve back pressure regulator that could be set using a given pressure of nitrogen.

UHPLC/UV analysis on lignin-derived monomers and oligomers was obtained on a Waters Acquity ultra-performance liquid chromatography (UPLC) unit equipped with a diode array detector and a Waters Acquity UPLC BEH C18 (50 mm x 2.3 mm ID -1.7 micron particle size) at 35 °C. Solvent A was 0.1% formic acid type 1 water and solvent B was HPLC grade acetonitrile with 0.1% formic acid (flow rate 1.75 mL/min). Monomer yields were quantified by making a calibration curve with each of the commercially available products using a 10 mM 1,4-dimethoxybenzene internal standard.

### *Lignin Depolymerization in Flow*

In a typical reaction, a 250 mL stock solution of 0.5-1 wt% lignin (1.25-2.5 g) was made with 2M NaOH. $CuSO_4 \cdot 5H_2O$ (0.054 g) was dissolved in 1 mL of Type 1 water and added while agitating the stock solution. The stock solution was then sonicated to dissolve the lignin. Meanwhile the flow reactor was set to the desired temperature (175-210 °C) and flow rate (0.05 mL/min - 9.5mL/min) and allowed to come to temperature. The Parr vessel shell was pressurized to the desired pressure (25-100 psig (172-689 kPag)) with gas (oxygen, nitrogen, or air). The back pressure regulator was set to 200 psig (1379 kPag) higher than pressure in the Parr vessel shell. Once the temperature was equilibrated, the pump feed was changed to pull from the lignin stock solution. The lignin solution was allowed to run for 5 residence times before an aliquot was taken for sampling. A 0.5 mL aliquot taken for sampling mixture was mixed with a 10 mM 1,4-dimethoxybenzene stock solution used as an internal standard and then acidified to below pH 2 with HCl. The depolymerized mixture was filtered (0.22-micron PTFE) prior to analysis by UPLC.

### *$CuSO_4$ Lignin Oxidation in Batch*

$CuSO_4$ oxidations were carried out similar to other reports in the literature. In brief, 50 mg of lignin was added into a small PTFE cup (Max volume of 25 mL) along with $CuSO_4 \cdot 5H_2O$ (4 mg). Then 10 mL of 2 M NaOH solution made with Type 1 water was added to the cups along with a stir bar (PTFE, length x Dia. 7 mm X 2 mm). Up to 8 cups were placed in a 1L Parr vessel with 150 mL of water to assist in heat transfer. The Parr vessel was sealed and pressurized with air to 365 psig (2517 kPag). The reaction was heated to 175 °C using a tuned Parr 4838 temperature controller with a K type thermocouple. The reaction time was started after turning on the temperature controller. Once the reaction reached the desired time, the reactor was removed from the heater and placed in an ice bath until the reactor had returned to room temperature. A 0.5 mL aliquot taken for sampling mixture was mixed with a 10 mM 1,4-dimethoxybenzene stock solution used as an internal standard and then acidified to pH 2 with HCl. The depolymerized mixture was filtered (0.22-micron PTFE) prior to analysis by UPLC.

### *CuO Lignin Oxidation in Batch*

CuO oxidations were carried out as in other reports in the literature. In brief, 50 mg of lignin was added into a small PTFE cup (Max volume of 25 mL) along with CuO (300 mg). Then 10 mL of sparged 2 M NaOH aqueous solution made with Type 1 water was added to the cups along with a stir bar (PTFE, length x Dia. 7 mm X 2 mm). Up to 8 cups were placed in a 1 L Parr vessel with 150 mL of water to assist in heat transfer. The Parr vessel was sealed and pressurized with nitrogen while recycling 5 times to remove advantageous oxygen before being pressurized to 365 psig (2517 kPag). The reaction was heated to 180 °C using a tuned Parr 4838 temperature controller with a K type thermocouple. The reaction time was started after turning on the temperature controller. The reaction was allowed to proceed for 130 minutes before being removed from the heater and placed in an ice bath until the reactor had returned to room temperature. A 0.5 mL aliquot taken for sampling mixture was mixed with a 10 mM 1,4-dimethoxybenzene stock solution used as an internal standard and then acidified to pH 2 with HCl. The depolymerized mixture was filtered (0.22-micron PTFE) prior to analysis by UPLC.

### *Lignin Hydrogenolysis in Batch*

In a typical reaction 50 mg of lignin and ruthenium on carbon (5wt/wt%, 25 mg), was placed in a Hastelloy steel

Parr vessel with a PTFE coated stir bar. Then 5 mL of tetrahydrofuran solvent was added. The headspace was purged 3 times with hydrogen gas (580 psig (3999 kPag)). The reactor was heated to 250 °C and held for 3 hours while stirring. The reactor was cooled to room temperature and depressurized. Internal standard was added (0.2 mL of n-decane) and then 1 mL was removed filtered (0.22 micron PTFE filter) prior to analysis by gas chromatography.

***Barrer Solubility Test***

The flow reactor was set to the desired temperature (210 °C) and flow rate (0.05 mL/min-9.5mL/min) and allowed to come to temperature. The Parr vessel shell was pressurized to the desired pressure (25-100 psig (172-689 kPag)) with oxygen gas. The back pressure regulator was set to 200 psi (1379 kPa) higher than pressure in the Parr vessel shell. Once the temperature was equilibrated, the pump feed was changed to pull from a solution of 2 M NaOH. The solution was allowed to run for 5 residence times before the volume of gas and liquid were measured exiting the reactor using an upside down graduated cylinder submerged in a water bath which would fill up with oxygen. The reactor was set to a new flow rate and the analysis was repeated.

**Table 1.** Single tube reactor materials.

| Part Description | Supplier | Number of components |
|---|---|---|
| **HPLC pump** | | |
| L-6200 Intelligent HPLC pump | Hitachi | 1 |
| **Reactor shell and Oxygen purge** | | |
| 600 mL Parr reactor with two 1/4 in. (6 mm) Male NPT ports drilled in the bottom | Parr Instrument Company | 1 |
| Heater and temperature controller | Parr Instrument Company | 1 |
| Glass body with 316 stainless steel rotameter, 0-50cc/min, 1/8 FNPT | McMaster-Carr | 1 |
| Stainless steel tube fitting, union tee, 1/8 in. (3 mm) tube OD | Swagelok | 1 |
| Stainless steel back pressure regulator, FKM seat, 1/4 in. (6 mm) FNPT, 0.20 CV | Swagelok | 1 |
| Stainless steel instrumentation quick connect body, 0.08 Cv, 1/8 in. (3 mm) tube fitting | Swagelok | 1 |
| Stainless steel quarter turn instrument plug valve, 1/8 in. (3 mm) Swagelok tube fitting, 0.10 Cv, black handle | Swagelok | 1 |
| Single scale pressure gauge with 304 stainless steel case, 1/8 NPT male center back connection, 1-1/2" (4 cm) dial | Swagelok | 1 |
| **Back pressure regulator set up** | | |
| Back pressure regulator, dome valve | Similar to ones from Equilibar LLC | 1 |
| Stainless steel tube fitting, male connector, 1/16 in. (1.6 mm)) tube OD x 1/4 in. (6 mm) male NPT | Swagelok | 1 |
| Stainless steel Swagelok tube fitting, male elbow, 1/8 in. (3 mm) tube OD x 1/8 in. (3 mm) male NPT | Swagelok | 1 |
| Stainless steel tube fitting, male connector, 1/16 in. (1.6 mm) tube OD x 1/4 in. (6 mm) male NPT | Swagelok | 1 |
| Stainless steel instrumentation quick connect body, 0.08 Cv, 1/8 in. (3 mm) tube fitting | Swagelok | 1 |
| Stainless steel quarter turn instrument plug valve, 1/8 in. (3 mm) Swagelok tube fitting, 0.10 Cv, black handle | Swagelok | 1 |

(continued)

| | | |
|---|---|---|
| **Back pressure regulator set up** | | |
| Stainless steel 1-piece 40G series 3-way ball valve, 0.15 Cv, 1/8 in. (3 mm) Swagelok tube fitting, | Swagelok | 1 |
| Stainless steel Swagelok tube fitting, male connector, 1/8 in. (3 mm) tube OD x 1/4 in. (6 mm) male NPT | Swagelok | 1 |
| Single scale pressure gauge with 304 stainless steel case, 1/8 NPT male center back connection, 1-1/2" (4 cm) dial | McMaster-Carr | 1 |
| **Tube side reactor** | | |
| Stainless Steel Tube Fitting, Bored-Through Male Connector, 1/8 in. (3 mm) Tube OD x 1/4 in. (6 mm) Male NPT | Swagelok | 2 |
| Stainless steel tube fitting, bored-through male connector, 1/16 in. (1.6 mm) tube OD x 1/4 in. (6 mm) male NPT | Swagelok | 2 |
| Stainless steel 1/16 in. (1.6 mm) ferrule set | Swagelok | |
| Stainless steel 1/8 in. (3 mm) ferrule set | Swagelok | |
| Stainless steel tube fitting, reducing union, 1/8 in. (3 mm) x 1/16 in. (1.6 mm) tube OD | Swagelok | 1 |
| Stainless steel tube fitting, union, 1/8 in. (3 mm) tube OD | Swagelok | 1 |
| Stainless steel tube fitting, union, 1/16 in. (1.6 mm) tube OD | Swagelok | 1 |
| PTFE Tubing ID X OD X Length 0.058 in. (1.6 mm) X 1/8 in. (3 mm) X 50 feet (15 m) | Sigma Aldrich | 1 |
| PTFE Tubing ID X OD X Length 0.031 in. (0.8 mm) X 1/16 in. (1.6 mm) X 50 feet (15 m) | Sigma Aldrich | 1 |
| PTFE Tubing ID X OD X Length 0.019 in. (0.5 mm) X 1/16 in. (1.6 mm) X 50 feet (15 m) | Sigma Aldrich | 1 |
| PTFE Tubing ID X OD X Length 0.010 in. (0.25 mm) X 1/16 in. (1.6 mm) X 50 feet (15 m) | Sigma Aldrich | 1 |

**Results**

The permeable membrane flow reactor was constructed as shown in FIG. 1, panel (a), using a stainless-steel Parr shell with 20 feet (6 m) of gas permeable membrane tubing inside. The stainless-steel shell could be pressurized with oxygen gas, air, or an inert gas. A gas purge system regulated by a back pressure regulator was constructed to maintain a constant pressure of the chosen gas throughout the duration of the reaction. A HPLC pump was used to pump in a liquid solution of lignin and copper catalyst. Upon exiting the reactor, the feed was cooled to room temperature in a water bath. The pressure of the liquid feed was controlled using a back pressure regulator with the pressure set to avoid vaporization of the liquid feed. (Typically the back pressure regulator was set to 250 psig (1724 kPag) to maintain a liquid stream.) PTFE tubing was throughout the reactor, and no stainless-steel components were in contact with the harsh basic conditions, which have been known to etch or otherwise damage steel at high temperatures.

We evaluated different types of inexpensive tubing which would be compatible with strongly basic conditions, temperatures more than 160 °C, and moderate pressures (<300 psig (<2068 kPag)). Only PTFE and Teflon™-PFA membranes were compatible with the criteria. Running a short time course of the aerobic lignin oxidation reaction showed that there was not much difference between the Teflon™ PFA and Teflon™ PTFE (FIG. 3). PTFE was chosen as it was easier and slightly more inexpensive to source.

Different tube diameters of PTFE tubing were tested for the aerobic lignin oxidation reaction (FIG.4, Tables 2-5). The time courses indicated that similar maximum monomer yields were achieved with tube diameters of 0.5 mm, 0.8 mm,

and 1.6 mm, with smaller tubes showing faster rates of reactivity (FIG. 4, Tables 2-4). For tubing that was much smaller (0.3 mm), the maximum lignin monomer yields were not achieved with the residence times achieved with our system (FIG. 4, Tables 4-5). Interestingly the phenol oxidation rate, which consumes the aromatics, appears to not be oxygen-diffusion limited. In order to maximize the amount of products produced, the larger 1.6 mm tubing was used for the rest of this study.

**Table 2.** Aerobic lignin oxidation with 1.6 mm ID tubing.

| | Major Phenolic Products wt% | | | | | | |
|---|---|---|---|---|---|---|---|
| Res. Time (s) | Vanillic acid | Syringic acid | Vanillin | Syringaldehyde | Acetovanillone | Acetosyringone | *p*HBA |
| 0 | 0 | 0 | 0.1 | 0.2 | 0 | 0 | 1.7 |
| 97 | 1.1 | 1.9 | 3.8 | 10.2 | 0.8 | 1.7 | 3.6 |
| 123 | 1.2 | 2.2 | 5.1 | 13.2 | 1.1 | 2.3 | 3.6 |
| 185 | 1.5 | 3.0 | 7.3 | 16.4 | 1.5 | 3.2 | 3.5 |
| 231 | 1.7 | 3.1 | 7.9 | 17.1 | 1.5 | 2.9 | 3.6 |
| 264 | 1.8 | 2.7 | 7.9 | 16.7 | 1.4 | 2.1 | 3.6 |
| 308 | 2.0 | 2.3 | 8.2 | 16.7 | 1.3 | 1.6 | 3.7 |
| 370 | 2.1 | 1.1 | 7.7 | 13.5 | 0.9 | 0.4 | 3.5 |

**Table 3.** Aerobic lignin oxidation with 0.8 mm ID tubing.

| | Major Phenolic Products wt% | | | | | | |
|---|---|---|---|---|---|---|---|
| Res. Time (s) | Vanillic acid | Syringic acid | Vanillin | Syringaldehyde | Acetovanillone | Acetosyringone | pHBA |
| 0 | 0.4 | 0.3 | 0.4 | 0.2 | 0 | 0 | 2.7 |
| 31 | 0.9 | 1.6 | 3.5 | 6.6 | 0.7 | 1.1 | 4.2 |
| 46 | 1.2 | 2.2 | 5.6 | 11.7 | 0.9 | 1.2 | 4.0 |
| 63 | 1.4 | 2.6 | 6.8 | 14.2 | 1.0 | 2.2 | 3.7 |
| 80 | 1.7 | 2.6 | 7.9 | 16.0 | 1.1 | 2.1 | 3.9 |
| 108 | 1.8 | 1.6 | 7.7 | 15.5 | 1.1 | 1.3 | 3.6 |
| 147 | 1.8 | 0.7 | 7.7 | 13.9 | 0.4 | 0.5 | 3.5 |
| 184 | 1.6 | 0.4 | 7.3 | 11.1 | 0.2 | 0.3 | 3.6 |

**Table 4.** Aerobic lignin oxidation with 0.5 mm ID tubing.

| | Major Phenolic Products wt% | | | | | | |
|---|---|---|---|---|---|---|---|
| Res. Time (s) | Vanillic acid | Syringic acid | Vanillin | Syringaldehyde | Acetovanillone | Acetosyringone | *p*HBA |
| 0 | 0.4 | 0.2 | 0.3 | 0.2 | 0 | 0 | 2.1 |
| 36 | 1.5 | 2.6 | 7.3 | 15.0 | 1.1 | 2.1 | 3.7 |
| 48 | 1.7 | 1.4 | 7.4 | 14.6 | 1.1 | 1.2 | 3.7 |
| 57 | 1.7 | 0.9 | 7.3 | 13.9 | 1.0 | 0.8 | 3.5 |
| 71 | 1.6 | 0.5 | 7.5 | 12.5 | 0.8 | 0.4 | 3.7 |
| 95 | 1.6 | 0.4 | 8.1 | 11.8 | 0.4 | 0.3 | 4.0 |
| 143 | 1.1 | 0 | 6.6 | 6.1 | 0.3 | 0.3 | 3.5 |
| 238 | 0.6 | 0 | 4.3 | 1.4 | 0.3 | 0.3 | 2.9 |

**Table 5.** Aerobic lignin oxidation with 0.3 mm ID tubing.

| | Major Phenolic Products wt% | | | | | | |
|---|---|---|---|---|---|---|---|
| Res. Time (s) | Vanillic acid | Syringic acid | Vanillin | Syringaldehyde | Acetovanillone | Acetosyringone | pHBA |
| 0 | 0.4 | 0.2 | 0.3 | 0.2 | 0 | 0 | 1.8 |
| 22 | 1.4 | 1.8 | 6.1 | 11.2 | 0.9 | 1.4 | 3.5 |
| 26 | 1.6 | 0.7 | 7.0 | 12.1 | 0.9 | 0.8 | 3.6 |
| 37 | 1.6 | 0.7 | 7.0 | 12.1 | 0.9 | 0.8 | 3.6 |
| 52 | 1.8 | 0.5 | 8.1 | 13.6 | 1.0 | 0.9 | 4.1 |

(continued)

| Major Phenolic Products wt% | | | | | | | |
|---|---|---|---|---|---|---|---|
| Res. Time (s) | Vanillic acid | Syringic acid | Vanillin | Syringaldehyde | Acetovanillone | Acetosyringone | pHBA |
| 86 | 1.6 | 0.4 | 8.6 | 11.5 | 0.9 | 0.6 | 4.4 |
| 129 | 1.1 | 0 | 7.1 | 6.5 | 0.6 | 0.3 | 3.8 |
| 258 | 0.8 | 0 | 4.6 | 2.0 | 0.4 | 0.2 | 3.0 |

Increasing the CuAHP Poplar lignin concentration from 0.5 wt% to 1.5 wt% showed that the reactions achieved similar maximum yields (FIG. 5). The rate of the production of the aromatics showed a possible saturation-type behavior for the lignin with very little difference in rates across these three concentrations.

Increasing the oxygen pressure on the shell-side of the reactor increases the reaction rate resulting in faster production of aromatics with very similar maximum yields of aromatics (38 wt%) (FIG. 6, Tables 6-8). Running at 75 psig (517 kPag) or higher, the fast reactivity made very precise process control necessary to avoid the rapid degradation of the aromatics. For the rest of the study, 50 psig (345 kPag) was be used to give additional time course information. Running the reaction with an inert gas resulted in yields of less than 10 wt% of aromatics that included p-hydroxybenzoic acid more than any other aromatic, as p-Hydroxybenzoic acid can be cleaved from lignin solely by hydrolysis of the *p*-hydroxybenzoate linkages appended to the lignin backbone (FIG. 6, Table 9). Small amounts of other aromatics were observed as well using nitrogen alone. This may be due to dissolved oxygen from the air in the solvent (see Table 9. Interestingly, once the aromatics were generated, they remained stable for residence times of more than 400 s.

**Table 6.** Aerobic lignin oxidation at 75 psig (kPag 517) $O_2$.

| Major Phenolic Products wt% | | | | | | | |
|---|---|---|---|---|---|---|---|
| Res. Time (s) | Vanillic acid | Syringic acid | Vanillin | Syringaldehyde | Acetovanillone | Acetosyringone | *p*HBA |
| 0 | 0 | 0 | 0.3 | 0.2 | 0 | 0 | 1.5 |
| 98 | 1.3 | 2.4 | 5.4 | 13.2 | 1.1 | 2.3 | 3.7 |
| 123 | 1.4 | 2.8 | 6.7 | 15.0 | 1.2 | 2.8 | 3.6 |
| 143 | 1.6 | 2.8 | 7.3 | 16.0 | 1.4 | 2.8 | 3.7 |
| 168 | 2.0 | 2.6 | 8.7 | 18.3 | 1.5 | 2.2 | 4.0 |
| 205 | 1.9 | 1.7 | 7.9 | 15.6 | 1.2 | 1.1 | 3.6 |
| 264 | 1.8 | 0.6 | 7.3 | 10.5 | 0.7 | 0 | 3.5 |
| 370 | 1.4 | 0.4 | 6.4 | 6.8 | 0.6 | 0 | 3.3 |

**Table 7.** Base Conditions: Flow aerobic alkaline lignin depolymerization using 1.6 mm ID PTFE tubing[a]

| Major Phenolic Products wt% | | | | | | | |
|---|---|---|---|---|---|---|---|
| Res. Time (s) | Vanillic acid | Syringic acid | Vanillin | Syringaldehyde | Acetovanillone | Acetosyringone | *p*HBA[b] |
| 0 | 0 | 0 | 0.1 | 0.2 | 0 | 0 | 1.7 |
| 97 | 1.1 | 1.9 | 3.8 | 10.2 | 0.8 | 1.7 | 3.6 |
| 123 | 1.2 | 2.2 | 5.1 | 13.2 | 1.1 | 2.3 | 3.6 |
| 185 | 1.5 | 3.0 | 7.3 | 16.4 | 1.5 | 3.2 | 3.5 |
| 231 | 1.7 | 3.1 | 7.9 | 17.1 | 1.5 | 2.9 | 3.6 |
| 264 | 1.8 | 2.7 | 7.9 | 16.7 | 1.4 | 2.1 | 3.6 |
| 308 | 2.0 | 2.3 | 8.2 | 16.7 | 1.3 | 1.6 | 3.7 |
| 370 | 2.1 | 1.1 | 7.7 | 13.5 | 0.9 | 0.4 | 3.5 |

**Table 8.** Aerobic lignin oxidation at 30 psig (207 kPag) $O_2$.

| Major Phenolic Products wt% | | | | | | | |
|---|---|---|---|---|---|---|---|
| Res. Time (s) | Vanillic acid | Syringic acid | Vanillin | Syringaldehyde | Acetovanillone | Acetosyringone | *p*HBA |
| 0 | 0 | 0 | 0.3 | 0.1 | 0 | 0 | 1.4 |

(continued)

| Major Phenolic Products wt% | | | | | | | |
|---|---|---|---|---|---|---|---|
| Res. Time (s) | Vanillic acid | Syringic acid | Vanillin | Syringaldehyde | Acetovanillone | Acetosyringone | *p*HBA |
| 97 | 0.9 | 1.6 | 2.5 | 7.2 | 0.6 | 1.3 | 3.5 |
| 123 | 1.1 | 1.8 | 3.4 | 9.8 | 0.9 | 1.7 | 3.6 |
| 205 | 1.3 | 2.6 | 6.2 | 15.2 | 1.4 | 3.2 | 3.8 |
| 264 | 1.3 | 2.8 | 6.8 | 16.1 | 1.5 | 3.5 | 3.6 |
| 308 | 1.4 | 3.0 | 7.1 | 16.1 | 1.5 | 3.5 | 3.6 |
| 370 | 1.6 | 3.3 | 7.6 | 16.6 | 1.6 | 3.3 | 3.6 |
| 411 | 2.0 | 2.0 | 8.3 | 14.6 | 1.4 | 3.8 | 3.8 |

**Table 9.** Aerobic lignin oxidation at 50 psig (345 kPag) $N_2$.

| Major Phenolic Products wt% | | | | | | | |
|---|---|---|---|---|---|---|---|
| Res. Time (s) | Vanillic acid | Syringic acid | Vanillin | Syringaldehyde | Acetovanillone | Acetosyringone | *p*HBA |
| 0 | 0 | 0 | 0.3 | 0.1 | 0 | 0 | 1.3 |
| 97 | 0.8 | 1.4 | 1.0 | 1.9 | 0.4 | 0.7 | 3.6 |
| 123 | 0.8 | 1.4 | 1.0 | 1.7 | 0.5 | 0.9 | 3.5 |
| 205 | 0.9 | 1.4 | 1.0 | 1.8 | 0.5 | 1.0 | 3.6 |
| 411 | 0.9 | 1.4 | 1.1 | 1.9 | 0.6 | 1.0 | 3.6 |

Decreasing yields were observed with decreasing temperature (FIG. 7, Tables 10-12), potentially due to ineffective cleavage of one of the linkages in lignin at lower temperatures. Lowering the temperatures to temperatures the same as those used for batch $CuSO_4$ oxidation (175 °C) gave comparable maximum yields (30.5 wt% for flow and 28.8 wt% for batch) (FIG. 7, Table 12). However, the yield was achieved much quicker even when accounting for the heat up in the batch reaction (230 s for flow and more than 900 s accounting for heat up time for batch). The phenol oxidation reaction in the reactor does seem to be temperature dependent (FIG. 7).

**Table 10.** Aerobic lignin oxidation at 200 °C.

| Major Phenolic Products wt% | | | | | | | |
|---|---|---|---|---|---|---|---|
| Res. Time (s) | Vanillic acid | Syringic acid | Vanillin | Syringaldehyde | Acetovanillone | Acetosyringone | *p*HBA |
| 0 | 0.4 | 0.3 | 0.3 | 0.2 | 0 | 0 | 2.3 |
| 123 | 1.1 | 2.1 | 4.3 | 11.4 | 1.0 | 2.1 | 3.7 |
| 185 | 1.4 | 2.8 | 6.6 | 15.7 | 1.3 | 3.1 | 3.8 |
| 231 | 1.6 | 3.0 | 7.2 | 16.4 | 1.4 | 3.1 | 3.8 |
| 264 | 1.8 | 2.7 | 7.4 | 16.4 | 1.3 | 2.4 | 3.8 |
| 308 | 1.9 | 2.5 | 7.7 | 16.7 | 1.3 | 1.9 | 3.9 |
| 369 | 2.0 | 1.1 | 7.3 | 14.8 | 1.1 | 0.9 | 3.7 |
| 410 | 2.0 | 0.8 | 6.9 | 12.8 | 0.9 | 0.4 | 3.6 |

**Table 11.** Aerobic lignin oxidation at 190 °C.

| Major Phenolic Products wt% | | | | | | | |
|---|---|---|---|---|---|---|---|
| Res. Time (s) | Vanillic acid | Syringic acid | Vanillin | Syringaldehyde | Acetovanillone | Acetosyringone | *p*HBA |
| 0 | 0.4 | 0.3 | 0.3 | 0.2 | 0 | 0 | 0.4 |
| 123 | 1.1 | 1.9 | 3.8 | 9.6 | 0.8 | 1.8 | 3.8 |
| 185 | 1.3 | 2.4 | 5.6 | 13.4 | 1.1 | 2.5 | 3.8 |
| 231 | 1.4 | 2.9 | 6.9 | 15.7 | 1.3 | 3.0 | 3.6 |
| 264 | 1.5 | 2.9 | 7.1 | 15.4 | 1.3 | 2.8 | 3.5 |
| 308 | 1.6 | 2.8 | 7.2 | 15.5 | 1.3 | 2.7 | 3.5 |
| 369 | 1.8 | 2.0 | 7.4 | 15.1 | 1.3 | 1.7 | 3.5 |

(continued)

| Major Phenolic Products wt% | | | | | | | |
|---|---|---|---|---|---|---|---|
| Res. Time (s) | Vanillic acid | Syringic acid | Vanillin | Syringaldehyde | Acetovanillone | Acetosyringone | *p*HBA |
| 410 | 1.9 | 1.4 | 7.4 | 14.1 | 1.2 | 11.6 | 3.5 |

**Table 12.** Aerobic lignin oxidation at 175 °C.

| Major Phenolic Products wt% | | | | | | | |
|---|---|---|---|---|---|---|---|
| Res. Time (s) | Vanillic acid | Syringic acid | Vanillin | Syringaldehyde | Acetovanillone | Acetosyringone | pHBA |
| 0 | 0.4 | 0.3 | 0.3 | 0.2 | 0 | 0 | 2.4 |
| 123 | 0.8 | 1.4 | 2.8 | 5.4 | 0.6 | 1.1 | 3.6 |
| 185 | 1.1 | 2.0 | 4.3 | 9.2 | 0.9 | 1.8 | 3.6 |
| 231 | 1.4 | 2.7 | 6.0 | 12.9 | 1.1 | 2.4 | 4.0 |
| 264 | 1.3 | 2.5 | 5.8 | 12.2 | 1.1 | 2.2 | 3.7 |
| 308 | 1.4 | 2.6 | 6.1 | 12.4 | 1.1 | 2.2 | 3.6 |
| 369 | 1.5 | 2.4 | 6.5 | 12.7 | 1.1 | 2.0 | 3.5 |
| 410 | 1.6 | 2.1 | 6.5 | 12.3 | 1.1 | 1.8 | 3.4 |

Higher yields of aromatics were achieved by increasing the copper concentration from 0.8 mM to 1.6 mM (FIG. 8). Higher concentrations of copper salts were not possible in the conditions tested due to the insolubility of these copper species in sodium hydroxide. The reaction proceeded without the addition of a copper catalyst, but lower yields were achieved under these conditions. In addition to the rate of monomer production from lignin, the phenol oxidation reaction appeared to increase with increasing copper loading, resulting in increased consumption of the aromatics (FIG. 8). Using aldehyde model compounds (vanillin and syringaldehyde), phenol oxidation proceeded without oxidation of the aldehyde to carboxylic acid unless a copper catalyst was added.

To compare the reactivity in the flow reactor to batch reactivity or stoichiometric copper activity, lignins from a variety of different tree sources (birch, poplar, and pine) and using a variety of different isolation methods CuAHP method, Kraft, Lignosulfonate Calcium salt, and Glyoxylic acid aldehyde-protected lignin (Bertella et al. 2022) were tested FIGS. 9A and 9B). All lignin sources and species had similar reaction profiles. However, the phenol oxidation reaction was much slower in pine lignin due to the lower amount of electron-rich phenols. The CuAHP Poplar achieved yields of 38 wt% in flow and 29 wt% in batch and 37 wt% with super-stoichiometric CuO. Using GVL lignin resulted in 27 wt% in flow, 22 wt% in batch and 25 wt% in Super stoichiometric CuO. Results with the glyoxylic acid protected birch lignin resulted in 33 wt% yields for the flow oxidation and 21 wt% yield in batch and 31 wt% with super-stoichiometric CuO. The lower yields observed is likely due to the lack of easily removed p-hydroxybenzoate linkages in birch woods. CuAHP pine lignin resulted in 16 wt% yield of aromatic monomers in flow, 5 wt% aromatics in batch, and 9 wt% aromatics using super-stoichiometric metal salts. Glyoxylic acid protected pine lignin resulted in yields of 16 wt% for the flow oxidation, 10 wt% in the batch reactor and 11 for the super-stoichiometric CuO. Soft-wood Kraft lignin resulted in 10 wt% yield in flow, 6 wt% in batch and 11 wt% using super-stoichiometric metal salts.

Each of these lignin samples represented a different amount of the total lignin extracted based on Klason lignin content from the original biomass. For the hardwood samples, CuAHP was able to extract 35 wt% of the lignin, GVL was able to extract 28 wt% of the lignin, and glyoxylic acid was able to extract 78 wt% based on lignin. For the softwood samples CuAHP was able to extract 15 wt% of the lignin and glyoxylic acid was able to extract 76 wt% of the lignin. An accurate extraction yield could not be determined for the Kraft sample. For the Glyoxylic acid protected birch and pine samples direct comparison of this method to reductive methods could be done. Results showed that 71.1 g aromatics $\times$ $kg^{-1}$ biomass or 397 $\mu$mol $g^{-1}$ biomass could be valorized using this method and 52.5 g aromatics $\times$ $kg^{-1}$ biomass or 282 $\mu$mol $g^{-1}$ could be achieved using hydrogenolysis of birch lignin. Using glyoxylic acid protected pine lignin 40.0 g aromatics $\times$ $kg^{-1}$ biomass or 255 $\mu$mol $g^{-1}$ biomass could be achieved using the flow method and 20.2 g aromatics $\times$ $kg^{-1}$ biomass or 121 $\mu$mol $g^{-1}$ biomass could be achieved using hydrogenolysis.

Simple kinetic modelling was performed using experimentally determined diffusion and reaction constants (FIGS. 10A and 10B). The diffusion rate and oxygen saturation rate were found experimentally to be 4 barrer and 0.2 mM respectively. The phenol oxidation mechanism was found experimentally. This was put into a simple model using the equations in Eq. 1. The $k_3$ was floated to match the data, as was the maximum amount of converted lignin. The fit showed good agreement with experimental results and indicated that there is a sharp increase in the amount of soluble oxygen once all the easily convertible lignin was consumed.

$$\frac{d[O_2]_{aq}}{d\tau} = k_1\left([O_2]_{sat,aq} - [O_2]_{aq}\right) - (k_2[lignin] + k_2[Ar])[O_2]_{aq}$$
$$\frac{d[lignin]}{d\tau} = -k_2[lignin][O_2]_{aq}$$
$$\frac{d[Ar]}{d\tau} = k_2[lignin][O_2]_{aq} - k_3[Ar][O_2]_{aq} \quad \text{(Eq. 1)}$$

Using the permeable reactor enabled the ability to shut down the phenol oxidation of the electron rich products, which has limited the maximum yield achieved in other instances of alkaline aerobic lignin depolymerizations. The permeable membrane limits the diffusion of oxygen (which is shown by the zero-order reactivity of lignin), the temperature-independent reactivity, and the dependence on the inner diameter of the tube on the reactivity of the lignin. Using a simple kinetic model with experimentally determined constants further confirmed that the improved reactivity could be attributed to the lack of phenol oxidation. It is therefore likely that the use of super-stoichiometric metal oxides such as CuO can serve as an oxidant to depolymerize lignin but are not potent enough to oxidize the phenol and cause loss of the products.

The usage of permeable membrane reactors such as the reactor exemplified herein open up the potential for higher yielding and less expensive processing of lignin compared to batch systems currently in operation. The additional control of the reaction has resulted in improvements like increased pressure and increased loading. The use of parallel tube reactors in the same shell could be used to achieve much greater amounts of aromatics produced from lignin in a continuous manner. In addition, the lower pressures used (50 psig (345 kPag) operating pressure for flow compared to 375 psig (2586 kPag) starting pressure for batch) would make processing lignin much safer and allow for less expensive reactors to be built to process lignin. Finally, because all the wetted materials are made from PTFE, the material incompatibility with hot alkaline conditions at pressure is no longer a concern, potentially saving substantial reactor costs.

REFERENCES

Ali, Md. E.; Rahman, Md. M.; Sarkar, S. M.; Hamid, S. B. A., Heterogeneous Metal Catalysts for Oxidation Reactions. Journal of Nanomaterials 2014, Article ID 192038, pp. 1-23.

Aráujo, J. D. P.; Grande, C. A. Rodrigues, A. E. Structured Packed Bubble Column Reactor for Continuous Vanillin Production from Kraft Lignin. Catal. Today, 2009, 147, S330-S335. DOI: 10.1016/j.cattod.2009.07.016

Bassett, A. W.; Honnig, A. E.; Breyta, C. M.; Dunn, I. C.; La Scala, J. J.; Stanzione, J. F., III Vanillin-Based Resin for Additive Manufacturing. ACS Sustainable Chem. Eng. 2020, 8, 5626-5635, DOI: 10.1021/acssuschemeng.0c00159

Beckham, G. T.; Johnson, C. W.; Karp, E. M.; Salvachia, D.; Vardon, D. R. Opportunities and Challenges in Biological Lignin Valorization. Curr. Opin. Biotechnol. 2016, 42, 40-53, DOI: 10.1016/j.copbio.2016.02.030

Bertella S, Bernardes Figueirêdo M, De Angelis G, Mourez M, Bourmaud C, Amstad E, Luterbacher JS. Extraction and Surfactant Properties of Glyoxylic Acid-Functionalized Lignin. ChemSusChem. 2022 Aug 5;15(15):e202200270. doi: 10.1002/cssc.202200270. Epub 2022 Jun 7.

Bjørsvik, H-R.; Minisci, F. Fine Chemicals from Lignosulfonates 1. Synthesis of Vanillin by Oxidation of Lignosulfonates. Org. Proc. Res. Dev. 1999, 3, 330-340. DOI: 10.1021/op9900028

Bosque, I.; Magallanes, G.; Rigoulet, M.; Kärkäs, M. D.; Stephenson, C. R. J. Redox Catalysis Facilitates Lignin Depolymerization. ACS Cent. Sci. 2017, 3, 621-628, DOI: 10.1021/acscentsci.7b00140

Cui, Y.; Goes, S. L.; Stahl, S. S. Sequential Oxidation - Depolymerization Strategies for Lignin Conversion to Low Molecular Weight Aromatic Chemicals. In Advances in Inorganic Chemistry: Catalysis in Biomass Conversion; Ford, P. C., van Eldik, R., Eds.; Academic Press: Cambridge, MA, 2021; Vol. 77, pp 99-136

Das, A.; Rahimi, A.; Ulbrich, A.; Alherech, M.; Motagamwala, A. H.; Bhalla, A.; Sousa, L. C.; Balan, V.; Dumesic, J. A.; Hegg, E. L.; Dale, B. E.; Ralph, J.; Coon, J. J.; Stahl, S. S. Lignin Conversion to Low-Molecular-Weight Aromatics via an Aerobic Oxidation-Hydrolysis Sequence: Comparison of Different Lignin Sources. ACS Sustainable Chem. Eng. 2018, 6, 3367-3374, DOI: 10.1021/acssuschemeng.7b03541

Du, X.; Tricker, A. W.; Yang, W.; Katahira, R.; Liu, W.; Kwok, T. T.; Gogoi, P.; Deng, Y. Oxidative Catalytic Fractionation and Depolymerization of Lignin in a One-Pot Single-Catalyst System. ACS Sustainable Chem. Eng. 2021, 9, 7719-7727, DOI: 10.1021/acssuschemeng.0c08448

Fang, W.; Sixta, H. Advanced biorefinery based on the fractionation of biomass in γ-valerolactone and water. ChemSusChem 2015, 8 (1), 73-76.

Fargues, C.; Mathias, A.; Silva, J.; Rodrigues, A. Kinetics of Vanillin Oxidation. Chem. Eng. Technol. 1996, 19, 127-136. DOI: 10.1002/ceat.270190206

Gelosia, M.; Ingles, D.; Pompili, E.; D'Antonio, S.; Cavalaglio, G.; Petrozzi, A.; Coccia, V. Fractionation of lignocellulosic residues coupling steam explosion and organosolv treatments using green solvent γ-valerolactone. Energies 2017, 10 (9), 1264.

Gewirth, A. A.; Varnell, J. A.; and DiAscro, A. M., Nonprecious Metal Catalysts for Oxygen Reduction in Heterogeneous Aqueous Systems. Chemical Reviews, 2018, 118, 2313-2339.

Giacobbe FW. Oxygen Permeability of Teflon-PFA Tubing. Journal of Applied Polymer Science. 1990, 39(5):1121-1132.

Greene, J. F.; Preger, Y.; Root, T. W. Stahl, S. S. Org. Process Res. Dev. 2015, 19, 7, 858.864. DOI: 10.1021/acs.oprd.5b00125

Gutmann, B.; Cantillo, D.; Kappe, C. O. Continuous Flow Technology - A Tool for the Safe Manufacturing of Active Pharmaceutical ingredients. Angew. Chem. Int. Ed. 2015, 54, 6688-6728. DOI: 10.1002/anie.201409318

Hocking, M. B. Vanillin: Synthetic Flavoring from Spent Sulfite Liquor. J. Chem. Educ. 1997, 74, 1055-1059, DOI: 10.1021/ed074p1055

Kaur, N. & Kishore, D. Catal Surv Asia 2013, 17 (1), 20-42.

Kumar AK and Sharma S. Recent Updates on Different Methods of Pretreatment of Lignocellulosic Feedstocks: A Review. Bioresour. Bioprocess. (2017) 4:7.

Kumar, P.; Barrett, D. M.; Delwiche, M. J.; Stroeve, P., Methods for Pretreatment of Lignocellulosic Biomass for Efficient Hydrolysis and Biofuel Production. Industrial & Engineering Chemistry Research 2009, 48, (8), 3713-3729.

Lancefield, C. S.; Ojo, O. S.; Tran, F.; Westwood, N. J. Isolation of Functionalized Phenolic Monomers through Selective Oxidation and C-O Bond Cleavage of the β-O-4 Linkages in Lignin. Angew. Chem., Int. Ed. 2015, 54, 258-262, DOI: 10.1002/anie.201409408

Langholtz, M. H.; Stokes, B. J.; Eaton, L. M. 2016 Billion-Ton Report: Advancing Domestic Resources for a Thriving Bioeconomy, Vol. 1: Economic Availability of Feedstocks; Technical report for U.S. Department of Energy, Oak Ridge National Laboratory: Oak Ridge, TN, July 2016.

Le, H. Q.; Ma, Y.; Borrega, M.; Sixta, H. Wood biorefinery based on γ-valerolactone/water fractionation. Green Chem. 2016, 18 (20), 5466-5476.

Le, H. Q.; Zaitseva, A.; Pokki, J. P.; Stahl, M.; Alopaeus, V.; Sixta, H. Solubility of organosolv lignin in γ-valerolactone/-water binary mixtures. ChemSusChem 2016, 9 (20), 2939- 2947.

Llevot, A.; Grau, E.; Carlotti, S.; Grelier, S.; Cramail, H. From Lignin-derived Aromatic Compounds to Novel Biobased Polymers. Macromol. Rapid Commun. 2016, 37, 9- 28, DOI: 10.1002/marc.201500474

Luo, H.; Weeda, E. P. Alherech, M. Anson, C. W.; Karlen, S. D.; Cui, Y.; Foster, C. E.; Stahl, S. S. J. Am. Chem. Soc. 2021, 143, 37, 15462-15470. DOI: 10.1021/jacs.1c08635

Luterbacher, J. S.; Rand, J. M.; Alonso, D. M.; Han, J.; Youngquist, J. T.; Maravelias, C. T.; Pfleger, B. F.; Dumesic, J. A. Nonenzymatic sugar production from biomass using biomass-derived γ-valerolactone. Science 2014, 343 (6168), 277-280.

Luterbacher, J. S.; Alonso, D. M.; Rand, J. M.; Questell-Santiago, Y. M.; Yeap, J. H.; Pfleger, B. F.; Dumesic, J. A. Solvent-enabled nonenyzmatic sugar production from biomass for chemical and biological upgrading. ChemSusChem 2015, 8 (8), 1317-1322.

Perez, J. M.; Kontur, W. S.; Alherech, M.; Coplien, J.; Karlen, S. D.; Stahl, S. S.; Donohue, T. J.; Noguera, D. R. Funneling Aromatic Products of Chemically Depolymerized Lignin into 2-Pyrone-4-6-Dicarboxylic Acid with Novosphingobium Aromaticivorans. Green Chem. 2019, 21, 1340-1350, DOI: 10.1039/C8GC03504K

Petersen, T. P.; Polyzos, A.; O'Brien, M, Ulven, T.; Baxendale, I. R.; Ley, S. V. The Oxygen-Mediated Synthesis of 1,3-Butadiynes in Continuous Flow: Using Teflon AF-2400 to Effect Gas/Liquid Contact. ChemSusChem 2012, 5, 274-277. DOI: 10.1002/cssc.201100339

Pinnau I, Toy LG. Gas and Vapor Transport Properties of Amorphous Perfluorinated Copolymer Memberanes Based on 2,2-Bistrifluoromethyl-4,5-difluoro-1,3-dioxole/tetrafluoroethylene. Journal of Membrane Science 1996, 109:125-133.

Rafiee, M.; Alherech, M.; Karlen, S. D.; Stahl, S. S. Electrochemical Aminoxyl-Mediated Oxidation of Primary Alcohols in Lignin to Carboxylic Acids: Polymer Modifications and Depolymerization. J. Am. Chem. Soc. 2019, 141, 15266-15276, DOI: 10.1021/jacs.9b07243

Roberts, V. M.; Stein, V.; Reiner, T.; Lemonidou, A.; Li, X.; Lercher, J. A. Towards Quantitative Catalytic Lignin Depolymerization. Chem. Eur. J. 2011, 17, 5939-5948. DOI: 10.1002/chem.201002438

Schutyser, W., Kruger, J.S., Robinson, A.M., Katahira, R., Brandner, D.G., Cleveland, N.S., Mittal, A., Peterson, D.J., Meilan, R., Román-Leshkov, Y., & Beckham, G.T. (2018). Revisiting alkaline aerobic lignin oxidation. Green Chemistry, 20, 3828-3844.

Srihar, P.; Araujo, J. D.; Rodrigues, A. Modeling of Vanillin Production in a Structured Bubble Column Reactor. Catal. Today, 2005, 105, 574-581. DOI: 10.1016/j.cattod.2005.06.044

Sun, T. Tian, B. Su, C. Recent advances in Fe (or Co)/N/C electrocatalysts for the oxygen reduction reaction in polymer

electrolyte membrane fuel cells. J. Mater. Chem. A. 5, 18933-18950 (2017).
Sun, Z., Fridrich, B., de Santi, A., Elangovan, S. & Barta, K. Bright Side of Lignin Depolymerization: Toward New Platform Chemicals. Chem. Rev. 118, 614-678 (2018).
Tuck, C. O.; Pérez, E.; Horváth, I. T.; Sheldon, R. A.; Poliakoff, M. Valorization of Biomass: Deriving More Value from Waste. Science 2012, 337, 695-699, DOI: 10.1126/science.1218930
Wu, M.; Liu, J.-K.; Yan, Z.-Y.; Wang, B.; Zhang, X.-M.; Xu, F.; Sun, R.-C. Efficient recovery and structural characterization of lignin from cotton stalk based on a biorefinery process using a γ-valerolactone/water system. RSC Adv. 2016, 6 (8), 6196-6204.
Xanthopoulou, E.; Terzopoulou, Z.; Zamboulis, A.; Papadopoulos, L.; Tsongas, K.; Tzetzis, D.; Papageorgiou, G. Z.; Bikiaris, D. N. Poly(Propylene Vanillate): A Sustainable Lignin-Based Semicrystalline Engineering Polyester. ACS Sustainable Chem. Eng. 2021, 9, 1383-1397, DOI: 10.1021/acssuschemeng.0c08346

## Claims

1. A method of depolymerizing lignin, the method comprising flowing an aqueous reaction medium comprising the lignin and a base through a lumen of a channel at a temperature and for a time effective to depolymerize at least a portion of the lignin to aromatic monomers, wherein the channel comprises an oxygen-permeable channel substrate comprising an inner surface facing the lumen and an outer surface facing away from the lumen, and wherein the outer surface of the oxygen-permeable channel substrate is in contact with oxygen gas.

2. The method of claim 1, wherein the oxygen-permeable channel substrate comprises a membrane.

3. The method of any prior claim, wherein the oxygen-permeable channel substrate comprises a polymeric membrane.

4. The method of any prior claim, wherein the oxygen-permeable channel substrate comprises a fluoropolymer membrane.

5. The method of any prior claim, wherein the oxygen-permeable channel substrate comprises any one or more of polytetrafluoroethylene, fluorinated ethylene propylene, perfluoroalkoxy alkane, and 2,2-bistrifluoromethyl-4,5-difluoro-1,3-dioxole/tetrafluoroethylene copolymer.

6. The method of any prior claim, wherein the channel is in the form of one or more tubes.

7. The method of any prior claim, wherein the medium comprises the lignin in an amount of 25% w/w or less.

8. The method of any prior claim, wherein the base comprises one or more of sodium hydroxide, lithium hydroxide, potassium hydroxide, rubidium hydroxide, cesium hydroxide, calcium hydroxide, strontium hydroxide, and barium hydroxide.

9. The method of any prior claim, wherein the medium comprises the base in an amount from 0.1 M to 4 M.

10. The method of any prior claim, wherein the reaction medium is flowed through the lumen of the channel in the presence of an oxidation catalyst.

11. The method of claim 10, wherein the oxidation catalyst comprises a metal-based catalyst.

12. The method of any one of claims 10-11, wherein the oxidation catalyst comprises one or more of a metal salt and a metal hydroxide.

13. The method of any one of claims 10-12, wherein the oxidation catalyst comprises one or more of copper, iron, cobalt, nickel, and manganese.

14. The method of any one of claims 10-13, wherein the reaction medium comprises the oxidation catalyst and the oxidation catalyst is flowed through the lumen of the channel as part of the reaction medium.

15. The method of any one of claims 10-14, wherein the reaction medium comprises the oxidation catalyst in an amount less than 2 mM.

16. The method of any prior claim, wherein the temperature is within a range from 150 °C to 250°C.

17. The method of any prior claim, wherein the temperature is within a range from 200 °C to 250 °C.

18. The method of any prior claim, wherein the oxygen gas is at a partial pressure of at least 10 psig (69 kPag).

19. The method of any prior claim, wherein the time is within a range from 5 seconds to 1,000 seconds.

20. The method of any prior claim, wherein the aromatic monomers comprise one or more of vanillin, syringaldehyde, *p*-hydroxybenzoic acid, vanillic acid, syringic acid, acetovanillone, and acetosyringone.

21. The method of any prior claim, wherein the flowing the reaction medium is conducted at least until the aromatic monomers are produced in an amount of at least 20% w/w of the lignin originally present in the reaction medium.

22. The method of claim 1, wherein:

the oxygen-permeable channel substrate comprises a polytetrafluoroethylene or perfluoroalkoxy alkane membrane;
the channel is in the form of one or more tubes;
the medium comprises the lignin in an amount of 25% w/w or less;
the base comprises one or more of sodium hydroxide, lithium hydroxide, potassium hydroxide, rubidium hydroxide, cesium hydroxide, calcium hydroxide, strontium hydroxide, and barium hydroxide;
the medium comprises the base in an amount from 0.1 M to 4 M;
the reaction medium is flowed through the lumen of the channel in the presence of an oxidation catalyst comprising one or more of copper, iron, cobalt, nickel, and manganese;
the reaction medium comprises the oxidation catalyst and the oxidation catalyst is flowed through the lumen of the channel as part of the reaction medium;
the reaction medium comprises the oxidation catalyst in an amount less than 2 mM;
the temperature is within a range from 200 °C to 250 °C;
the oxygen gas is at a partial pressure of at least 10 psig (69 kPag);
the time is within a range from 5 seconds to 1,000 seconds;
the aromatic monomers comprise one or more of vanillin, syringaldehyde, *p*-hydroxybenzoic acid, vanillic acid, syringic acid, acetovanillone, and acetosyringone; and
the flowing the reaction medium is conducted at least until the aromatic monomers are produced in an amount of at least 20% w/w of the lignin originally present in the reaction medium.

**Patentansprüche**

1. Verfahren zum Depolymerisieren von Lignin, das Verfahren umfassend ein Leiten eines wässrigen Reaktionsmediums, umfassend das Lignin und eine Base, durch ein Lumen eines Kanals bei einer Temperatur und für eine Zeit, die wirksam ist, um mindestens einen Teil des Lignins zu aromatischen Monomeren zu depolymerisieren, wobei der Kanal ein sauerstoffdurchlässiges Kanalsubstrat umfasst, umfassend eine Innenoberfläche, die dem Lumen zugewandt ist, und eine Außenoberfläche, die von dem Lumen abgewandt ist, und wobei die Außenoberfläche des sauerstoffdurchlässigen Kanalsubstrats in Kontakt mit Sauerstoffgas steht.

2. Verfahren nach Anspruch 1, wobei das sauerstoffdurchlässige Kanalsubstrat eine Membran umfasst.

3. Verfahren nach einem der vorstehenden Ansprüche, wobei das sauerstoffdurchlässige Kanalsubstrat eine polymere Membran umfasst.

4. Verfahren nach einem der vorstehenden Ansprüche, wobei das sauerstoffdurchlässige Kanalsubstrat eine Fluorpolymermembran umfasst.

5. Verfahren nach einem der vorstehenden Ansprüche, wobei das sauerstoffdurchlässige Kanalsubstrat eines oder mehrere von Polytetrafluorethylen, fluoriertem Ethylenpropylen, Perfluoralkoxyalkan und 2,2-Bistrifluormethyl-4,5-difluor-1,3-dioxol/Tetrafluorethylen-Copolymer umfasst.

6. Verfahren nach einem der vorstehenden Ansprüche, wobei der Kanal in der Form von einem oder mehreren Rohren vorliegt.

7. Verfahren nach einem der vorstehenden Ansprüche, wobei das Medium das Lignin in einer Menge von 25 Gew.-% oder weniger umfasst.

8. Verfahren nach einem der vorstehenden Ansprüche, wobei die Base eines oder mehrere von Natriumhydroxid, Lithiumhydroxid, Kaliumhydroxid, Rubidiumhydroxid, Cäsiumhydroxid, Calciumhydroxid, Strontiumhydroxid und Bariumhydroxid umfasst.

9. Verfahren nach einem der vorstehenden Ansprüche, wobei das Medium die Base in einer Menge von 0,1 M bis 4 M umfasst.

10. Verfahren nach einem der vorstehenden Ansprüche, wobei das Reaktionsmedium durch das Lumen des Kanals in der Gegenwart eines Oxidationskatalysators geleitet wird.

11. Verfahren nach Anspruch 10, wobei der Oxidationskatalysator einen metallbasierten Katalysator umfasst.

12. Verfahren nach einem der Ansprüche 10 bis 11, wobei der Oxidationskatalysator eines oder mehrere von einem Metallsalz und einem Metallhydroxid umfasst.

13. Verfahren nach einem der Ansprüche 10 bis 12, wobei der Oxidationskatalysator eines oder mehrere von Kupfer, Eisen, Kobalt, Nickel und Mangan umfasst.

14. Verfahren nach einem der Ansprüche 10 bis 13, wobei das Reaktionsmedium den Oxidationskatalysator umfasst und der Oxidationskatalysator als Teil des Reaktionsmediums durch das Lumen des Kanals geleitet wird.

15. Verfahren nach einem der Ansprüche 10 bis 14, wobei das Reaktionsmedium den Oxidationskatalysator in einer Menge von weniger als 2 mM umfasst.

16. Verfahren nach einem der vorstehenden Ansprüche, wobei die Temperatur innerhalb eines Bereichs von 150 °C bis 250 °C liegt.

17. Verfahren nach einem der vorstehenden Ansprüche, wobei die Temperatur innerhalb eines Bereichs von 200 °C bis 250 °C liegt.

18. Verfahren nach einem der vorstehenden Ansprüche, wobei das Sauerstoffgas bei einem Partialdruck von mindestens 10 psig (69 kPag) vorliegt.

19. Verfahren nach einem der vorhergehenden Ansprüche, wobei die Zeit innerhalb eines Bereichs von 5 Sekunden bis 1.000 Sekunden liegt.

20. Verfahren nach einem der vorstehenden Ansprüche, wobei die aromatischen Monomere eines oder mehrere von Vanillin, Syringaldehyd, p-Hydroxybenzoesäure, Vanillinsäure, Syringsäure, Acetovanillon und Acetosyringon umfassen.

21. Verfahren nach einem der vorstehenden Ansprüche, wobei das Leiten des Reaktionsmediums mindestens so lange durchgeführt wird, bis die aromatischen Monomere in einer Menge von mindestens 20 Gew.-% des ursprünglich in dem Reaktionsmedium vorhandenen Lignins erzeugt werden.

22. System nach Anspruch 1, wobei:

das sauerstoffdurchlässige Kanalsubstrat eine Polytetrafluorethylen- oder Perfluoralkoxyalkan-Membran umfasst;
der Kanal in Form eines oder mehrerer Rohre vorliegt;
das Medium das Lignin in einer Menge von 25 Gew.-% oder weniger umfasst;
die Base eines oder mehrere von Natriumhydroxid, Lithiumhydroxid, Kaliumhydroxid, Rubidiumhydroxid, Cäsiumhydroxid, Calciumhydroxid, Strontiumhydroxid und Bariumhydroxid umfasst;

das Medium die Base in einer Menge von 0,1 M bis 4 M umfasst;
das Reaktionsmedium durch das Lumen des Kanals in der Gegenwart eines Oxidationskatalysators geleitet wird, der eines oder mehrere von Kupfer, Eisen, Kobalt, Nickel und Mangan umfasst;
das Reaktionsmedium den Oxidationskatalysator umfasst und der Oxidationskatalysator als Teil des Reaktionsmediums durch das Lumen des Kanals geleitet wird;
das Reaktionsmedium den Oxidationskatalysator in einer Menge von weniger als 2 mM umfasst;
die Temperatur innerhalb eines Bereichs von 200 °C bis 250 °C liegt;
das Sauerstoffgas bei einem Partialdruck von mindestens 10 psig (69 kPag) vorliegt;
die Zeit innerhalb eines Bereichs von 5 Sekunden bis 1.000 Sekunden liegt;
die aromatischen Monomere eines oder mehrere von Vanillin, Syringaldehyd, *p*-Hydroxybenzoesäure, Vanillinsäure, Syringsäure, Acetovanillon und Acetosyringon umfassen; und
das Leiten des Reaktionsmediums mindestens so lange durchgeführt wird, bis die aromatischen Monomere in einer Menge von mindestens 20 Gew.-% des ursprünglich in dem Reaktionsmedium vorhandenen Lignins erzeugt werden.

## Revendications

**1.** Procédé de dépolymérisation de lignine, le procédé comprenant l’écoulement d’un milieu réactionnel aqueux comprenant la lignine et une base à travers une lumière d’un canal à une température et pendant un temps efficaces pour dépolymériser au moins une partie de la lignine en monomères aromatiques, dans lequel le canal comprend un substrat de canal perméable à l’oxygène comprenant une surface interne faisant face vers la lumière et une surface externe faisant face à l’écart de la lumière, et dans lequel la surface externe du substrat de canal perméable à l’oxygène est en contact avec de l’oxygène gazeux.

**2.** Procédé selon la revendication 1, dans lequel le substrat de canal perméable à l’oxygène comprend une membrane.

**3.** Procédé selon l’une quelconque revendication précédente, dans lequel le substrat de canal perméable à l’oxygène comprend une membrane en polymère.

**4.** Procédé selon l’une quelconque revendication précédente, dans lequel le substrat de canal perméable à l’oxygène comprend une membrane en fluoropolymère.

**5.** Procédé selon l’une quelconque revendication précédente, dans lequel le substrat de canal perméable à l’oxygène comprend l’un quelconque ou plusieurs quelconques parmi polytétrafluoroéthylène, éthylène-propylène fluoré, perfluoroalcoxy-alcane et copolymère 2,2-bistrifluorométhyl-4,5-difluoro-1,3-dioxole/tétrafluoroéthylène.

**6.** Procédé selon l’une quelconque revendication précédente, dans lequel le canal est sous la forme d’un ou plusieurs tubes.

**7.** Procédé selon l’une quelconque revendication précédente, dans lequel le milieu comprend la lignine en une quantité de 25 % en poids ou moins.

**8.** Procédé selon l’une quelconque revendication précédente, dans lequel la base comprend un ou plusieurs parmi hydroxyde de sodium, hydroxyde de lithium, hydroxyde de potassium, hydroxyde de rubidium, hydroxyde de césium, hydroxyde de calcium, hydroxyde de strontium et hydroxyde de baryum.

**9.** Procédé selon l’une quelconque revendication précédente, dans lequel le milieu comprend la base en une quantité allant de 0,1 M à 4 M.

**10.** Procédé selon l’une quelconque revendication précédente, dans lequel le milieu réactionnel est mis en écoulement à travers la lumière du canal en présence d’un catalyseur d’oxydation.

**11.** Procédé selon la revendication 10, dans lequel le catalyseur d’oxydation comprend un catalyseur à base de métal.

**12.** Procédé selon l’une quelconque des revendications 10 à 11, dans lequel le catalyseur d’oxydation comprend un ou plusieurs parmi un sel métallique et un hydroxyde métallique.

**13.** Procédé selon l'une quelconque des revendications 10 à 12, dans lequel le catalyseur d'oxydation comprend un ou plusieurs parmi cuivre, fer, cobalt, nickel et manganèse.

**14.** Procédé selon l'une quelconque des revendications 10 à 13, dans lequel le milieu réactionnel comprend le catalyseur d'oxydation et le catalyseur d'oxydation est mis en écoulement à travers la lumière du canal en tant que partie du milieu réactionnel.

**15.** Procédé selon l'une quelconque des revendications 10 à 14, dans lequel le milieu réactionnel comprend le catalyseur d'oxydation en une quantité inférieure à 2 mM.

**16.** Procédé selon l'une quelconque revendication précédente, dans lequel la température est au sein d'une plage allant de 150 °C à 250 °C.

**17.** Procédé selon l'une quelconque revendication précédente, dans lequel la température est au sein d'une plage allant de 200 °C à 250 °C.

**18.** Procédé selon l'une quelconque revendication précédente, dans lequel l'oxygène gazeux est à une pression partielle d'au moins 10 psig (69 kPag).

**19.** Procédé selon l'une quelconque revendication précédente, dans lequel le temps est au sein d'une plage allant de 5 secondes à 1000 secondes.

**20.** Procédé selon l'une quelconque revendication précédente, dans lequel les monomères aromatiques comprennent un ou plusieurs parmi vanilline, syringaldéhyde, acide p-hydroxybenzoïque, acide vanillique, acide syringique, acétovanillone et acétosyringone.

**21.** Procédé selon l'une quelconque revendication précédente, dans lequel l'écoulement du milieu réactionnel est effectué au moins jusqu'à ce que la monomères aromatiques soient produits en une quantité d'au moins 20 % en poids de la lignine initialement présente dans le milieu réactionnel.

**22.** Procédé selon la revendication 1, dans lequel :

le substrat de canal perméable à l'oxygène comprend une membrane en polytétrafluoroéthylène ou en perfluoroalcoxy-alcane ;
le canal est sous la forme d'un ou plusieurs tubes ;
le milieu comprend la lignine en une quantité de 25 % en poids ou moins ;
la base comprend un ou plusieurs parmi hydroxyde de sodium, hydroxyde de lithium, hydroxyde de potassium, hydroxyde de rubidium, hydroxyde de césium, hydroxyde de calcium, hydroxyde de strontium et hydroxyde de baryum ;
le milieu comprend la base en une quantité allant de 0,1 M à 4 M ;
le milieu réactionnel est mis en écoulement à travers la lumière du canal en présence de catalyseur d'oxydation comprenant un ou plusieurs parmi cuivre, fer, cobalt, nickel et manganèse ;
le milieu réactionnel comprend le catalyseur d'oxydation et le catalyseur d'oxydation est mis en écoulement à travers la lumière du canal en tant que partie du milieu réactionnel ;
le milieu réactionnel comprend le catalyseur d'oxydation en une quantité inférieure à 2 mM ;
la température est au sein d'une plage allant de 200 °C à 250 °C ;
l'oxygène gazeux est à une pression partielle d'au moins 10 psig (69 kPag) ;
le temps est au sein d'une plage allant de 5 secondes à 1000 secondes ;
les monomères aromatiques comprennent un ou plusieurs parmi vanilline, syringaldéhyde, acide *p*-hydroxybenzoïque, acide vanillique, acide syringique, acétovanillone et acétosyringone ; et
l'écoulement du milieu réactionnel est effectué au moins jusqu'à ce que les monomères aromatiques soient produits en une quantité d'au moins 20 % en poids de la lignine initialement présente dans le milieu réactionnel.

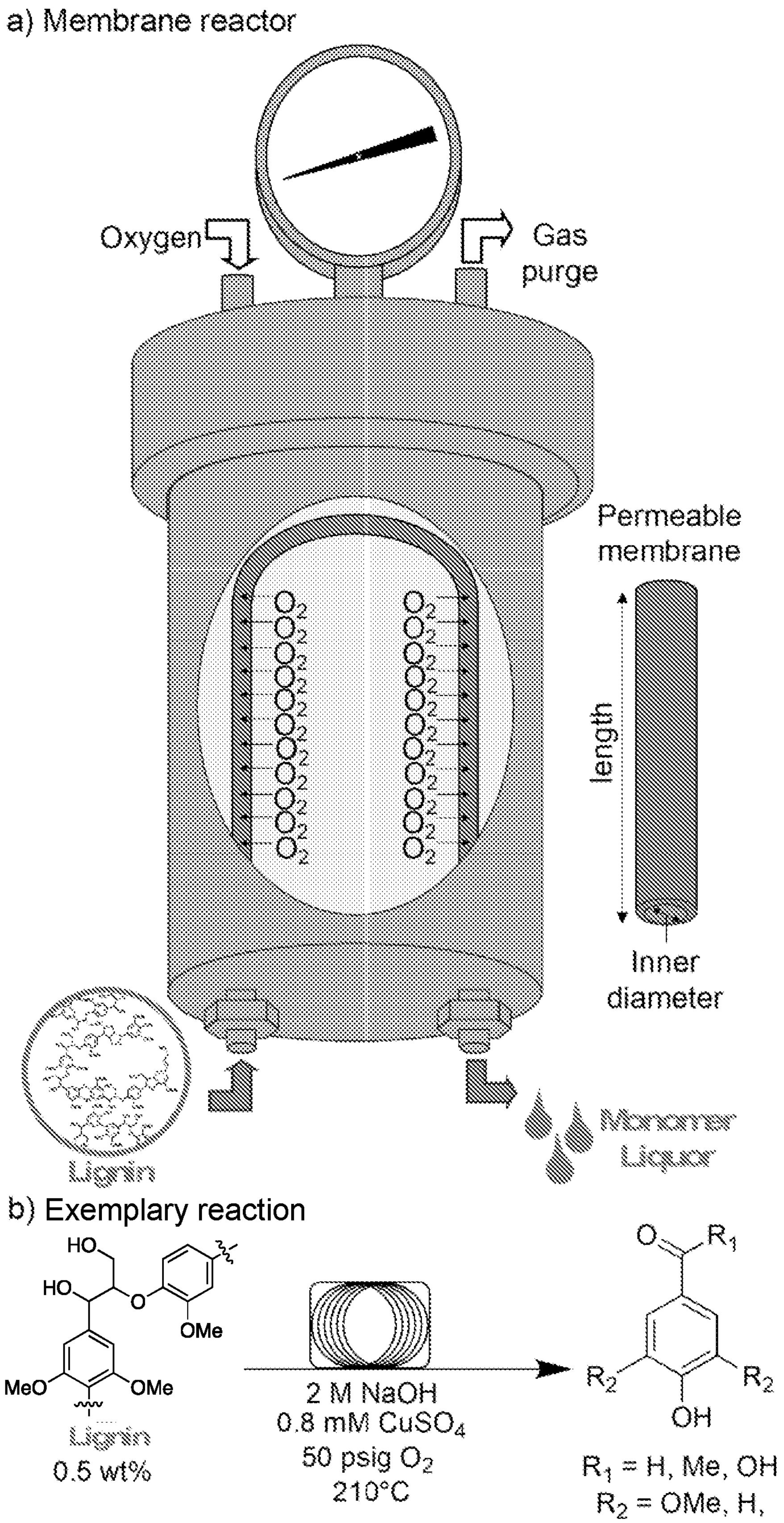
a) Membrane reactor
Oxygen
Gas purge
Permeable membrane
length
Inner diameter
Lignin
Monomer Liquor
b) Exemplary reaction
Lignin
0.5 wt%
2 M NaOH
0.8 mM $CuSO_4$
50 psig $O_2$
210°C
$R_1$ = H, Me, OH
$R_2$ = OMe, H,

**FIG. 1**

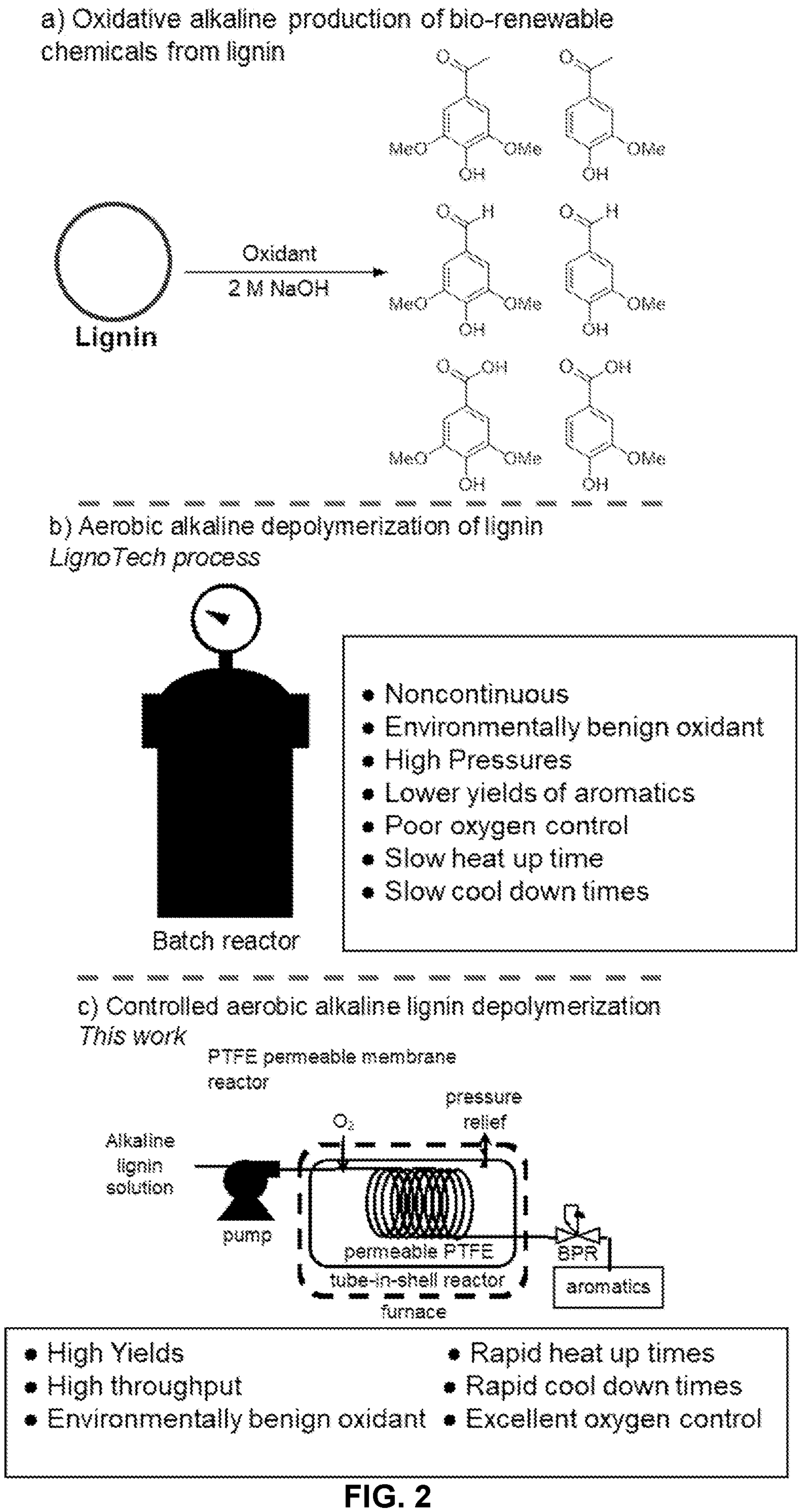
a) Oxidative alkaline production of bio-renewable chemicals from lignin
Oxidant
2 M NaOH
Lignin
b) Aerobic alkaline depolymerization of lignin
LignoTech process
Batch reactor
• Noncontinuous
• Environmentally benign oxidant
• High Pressures
• Lower yields of aromatics
• Poor oxygen control
• Slow heat up time
• Slow cool down times
c) Controlled aerobic alkaline lignin depolymerization
This work
PTFE permeable membrane reactor
Alkaline lignin solution
pump
pressure relief
permeable PTFE tube-in-shell reactor
furnace
BPR
aromatics
• High Yields
• High throughput
• Environmentally benign oxidant
• Rapid heat up times
• Rapid cool down times
• Excellent oxygen control

FIG. 2

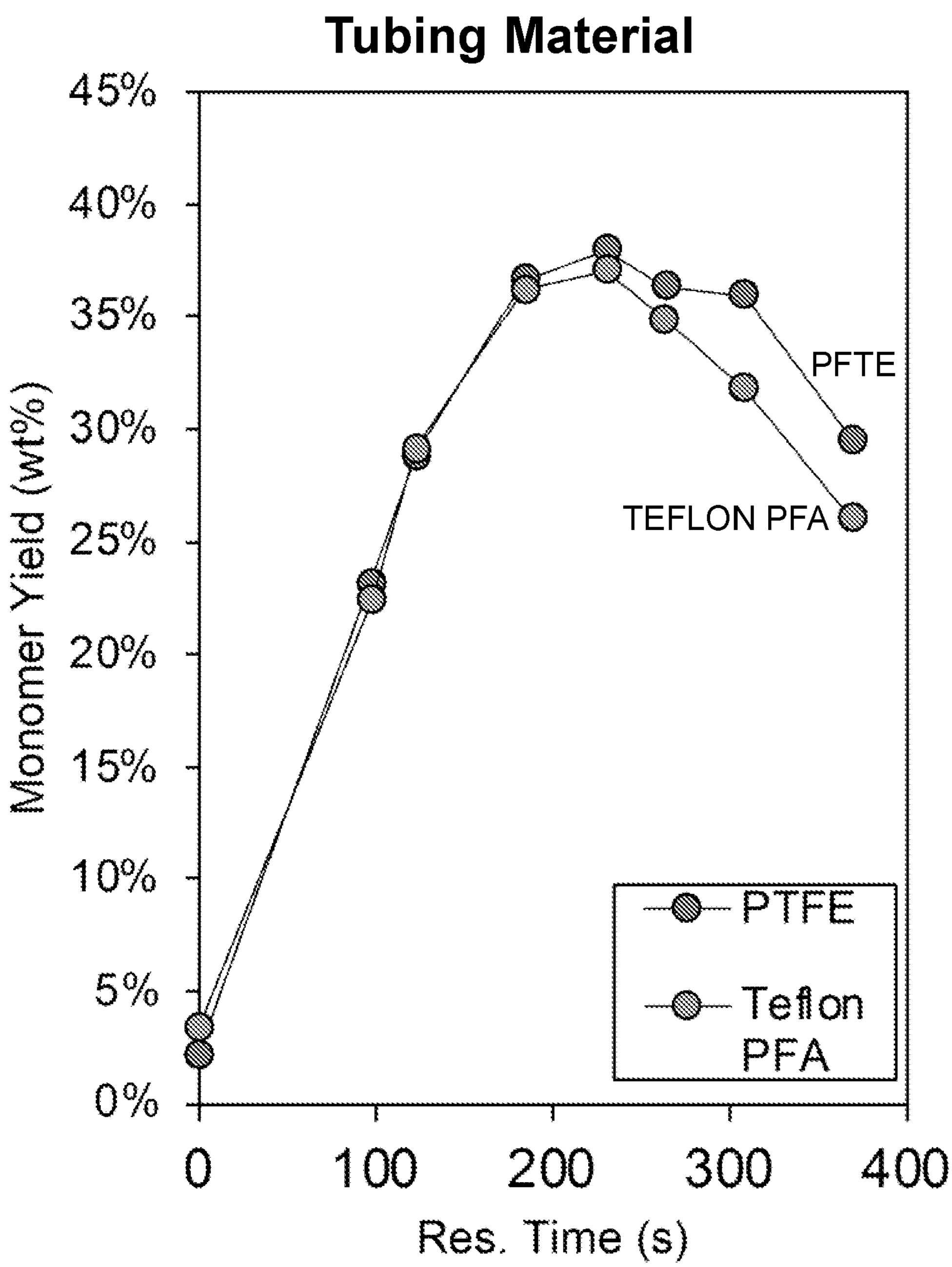
Tubing Material
45%
40%
35%
30%
25%
20%
15%
10%
5%
0%
Monomer Yield (wt%)
0
100
200
300
400
Res. Time (s)
PFTE
TEFLON PFA
PTFE
Teflon PFA

FIG. 3

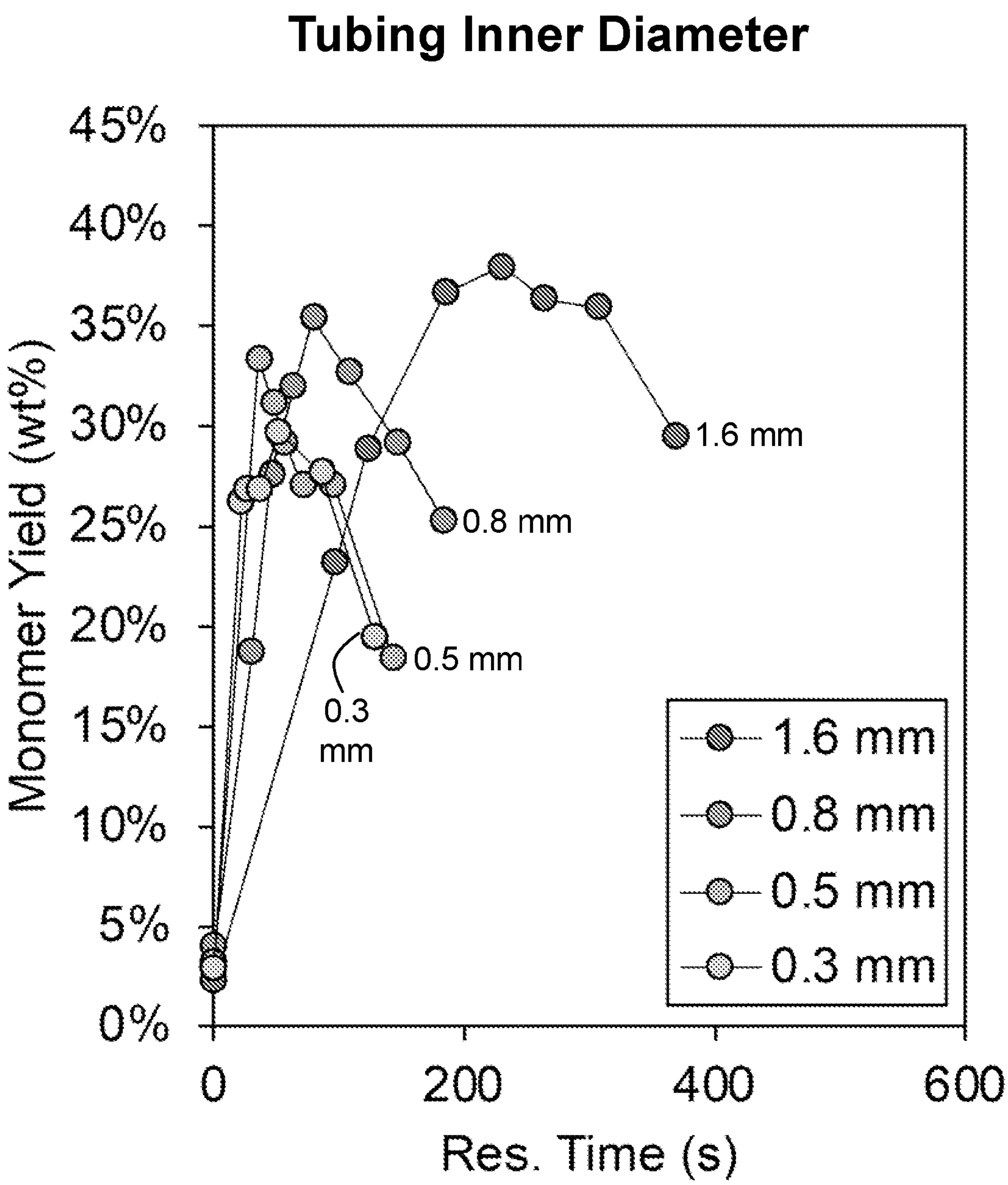
Tubing Inner Diameter
45%
40%
35%
30%
25%
20%
15%
10%
5%
0%
Monomer Yield (wt%)
0
200
400
600
Res. Time (s)
1.6 mm
0.8 mm
0.5 mm
0.3
mm
1.6 mm
0.8 mm
0.5 mm
0.3 mm

FIG. 4

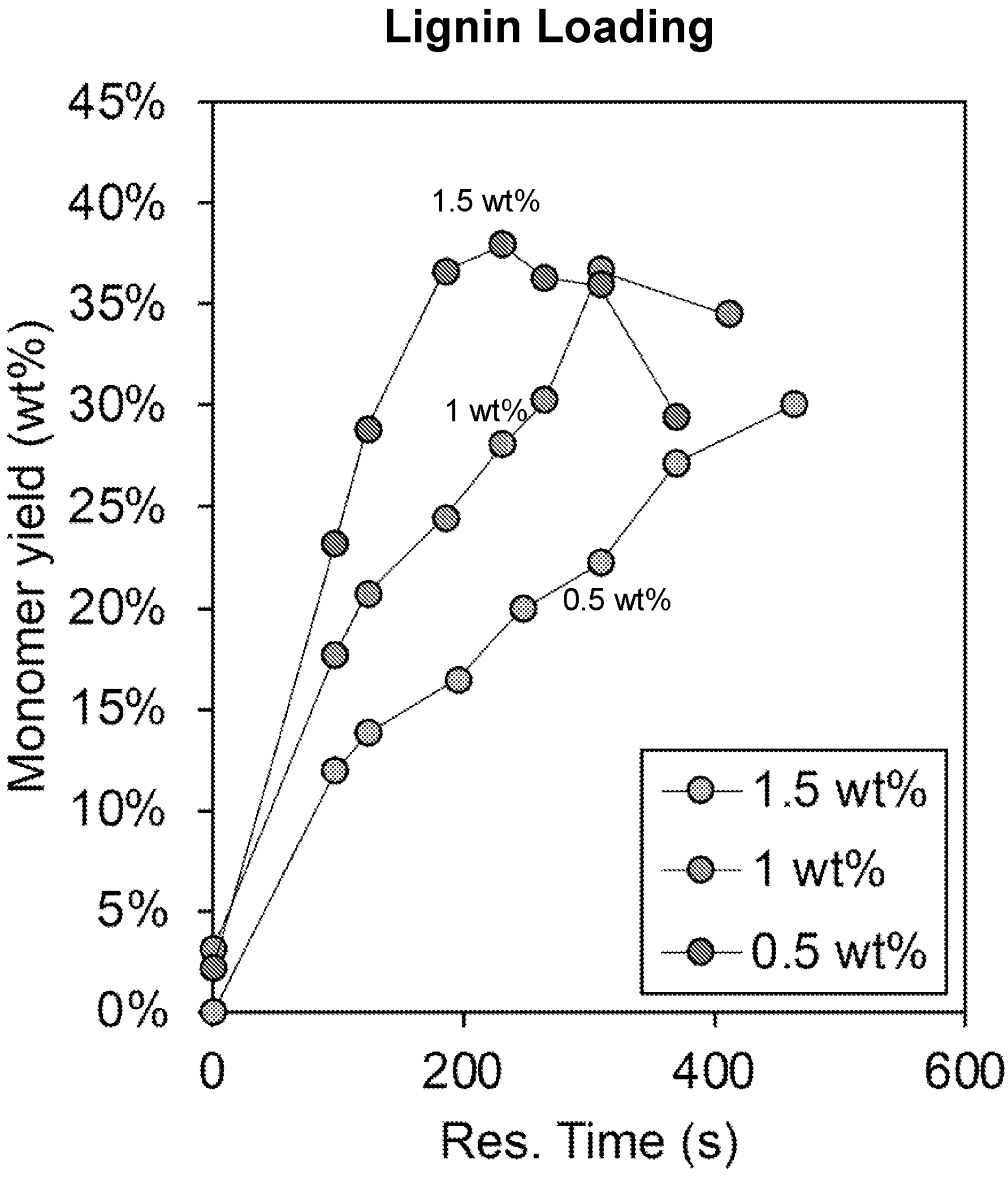
Lignin Loading
45%
40%
35%
30%
25%
20%
15%
10%
5%
0%
Monomer yield (wt%)
1.5 wt%
1 wt%
0.5 wt%
1.5 wt%
1 wt%
0.5 wt%
0
200
400
600
Res. Time (s)

FIG. 5

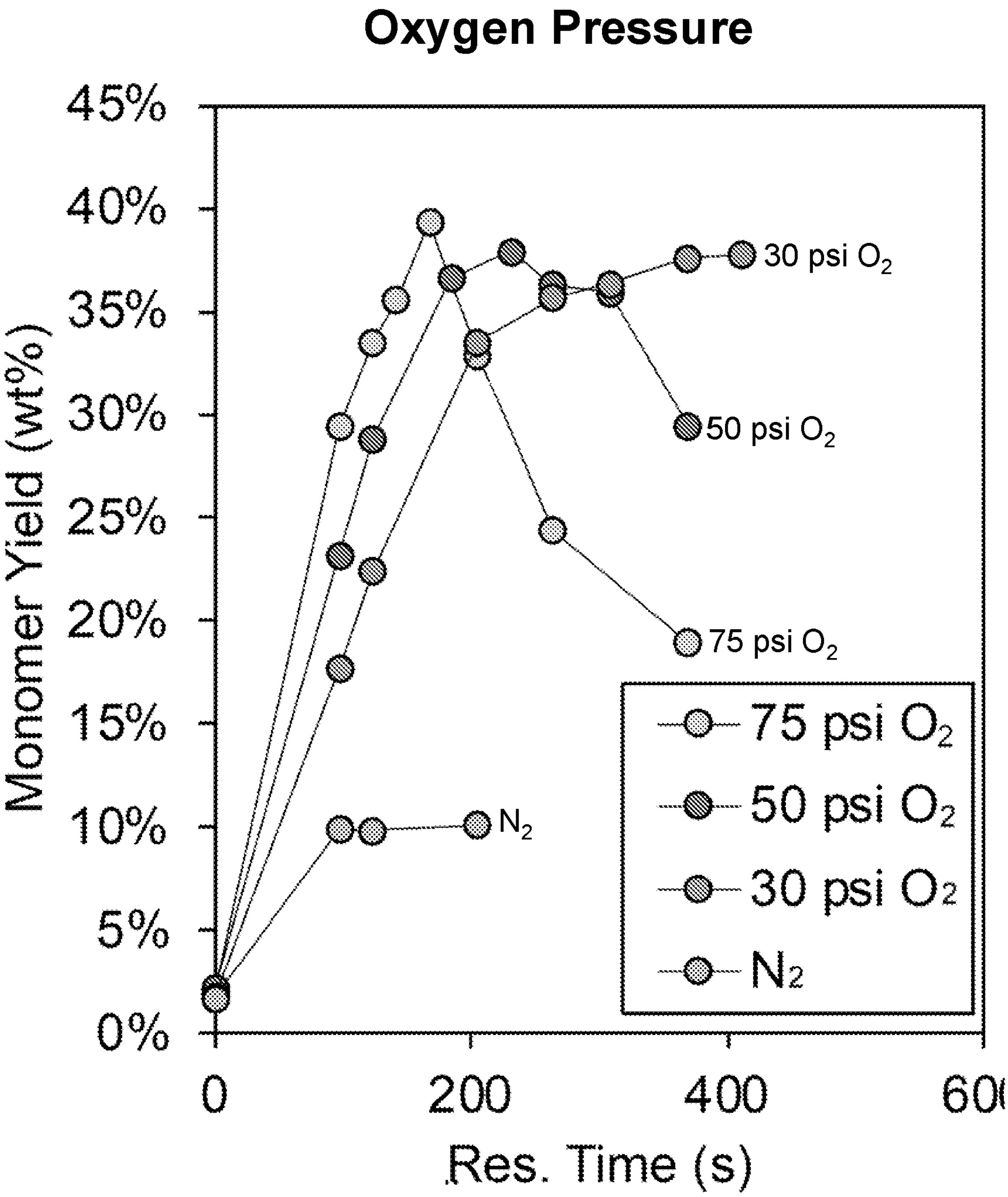
Oxygen Pressure
Monomer Yield (wt%)
45%
40%
35%
30%
25%
20%
15%
10%
5%
0%
0
200
400
60(
Res. Time (s)
30 psi $O_2$
50 psi $O_2$
75 psi $O_2$
$N_2$
75 psi $O_2$
50 psi $O_2$
30 psi $O_2$
$N_2$

FIG. 6

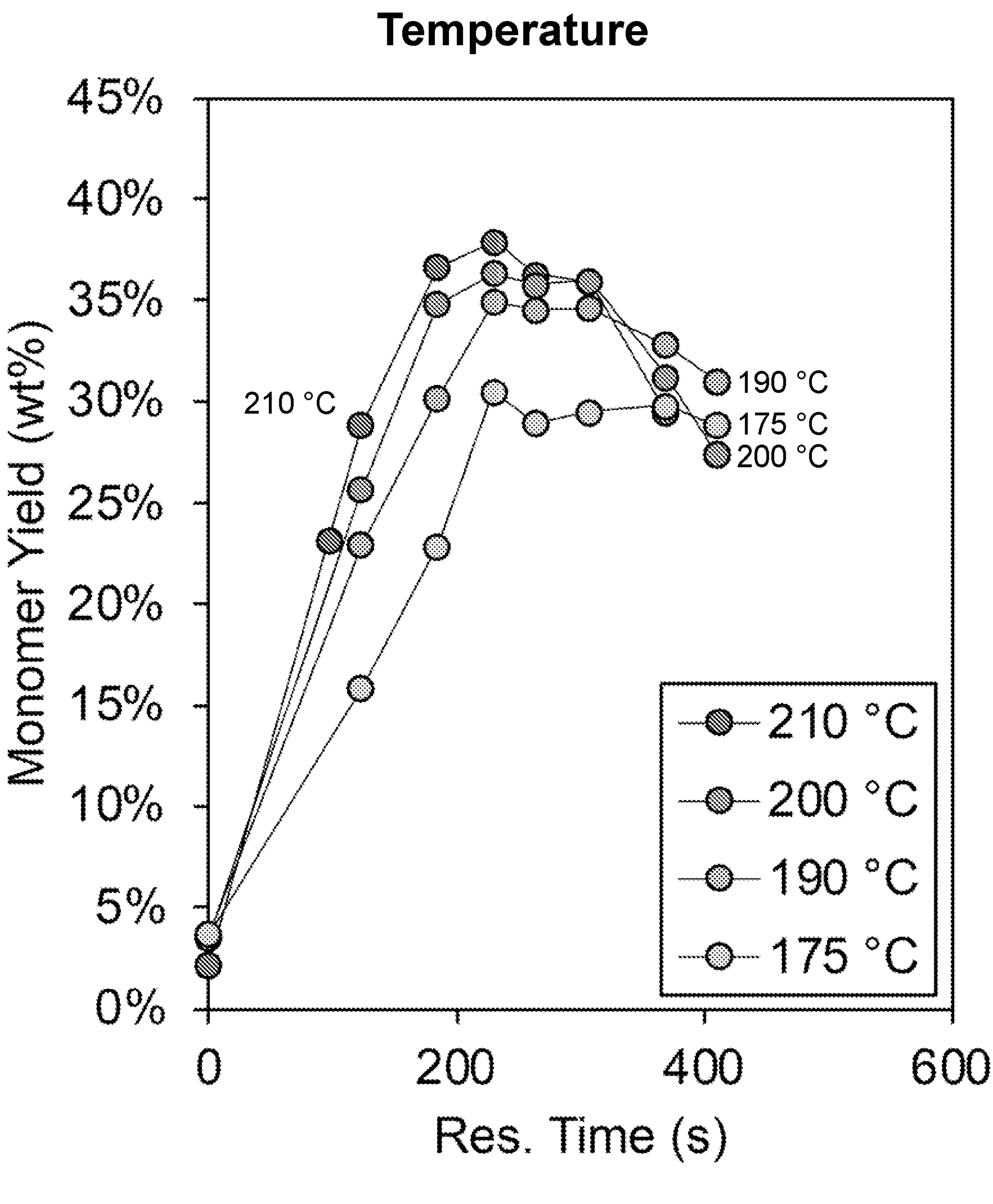
Temperature
Monomer Yield (wt%)
45%
40%
35%
30%
25%
20%
15%
10%
5%
0%
0
200
400
600
Res. Time (s)
210 °C
190 °C
175 °C
200 °C
210 °C
200 °C
190 °C
175 °C

**FIG. 7**

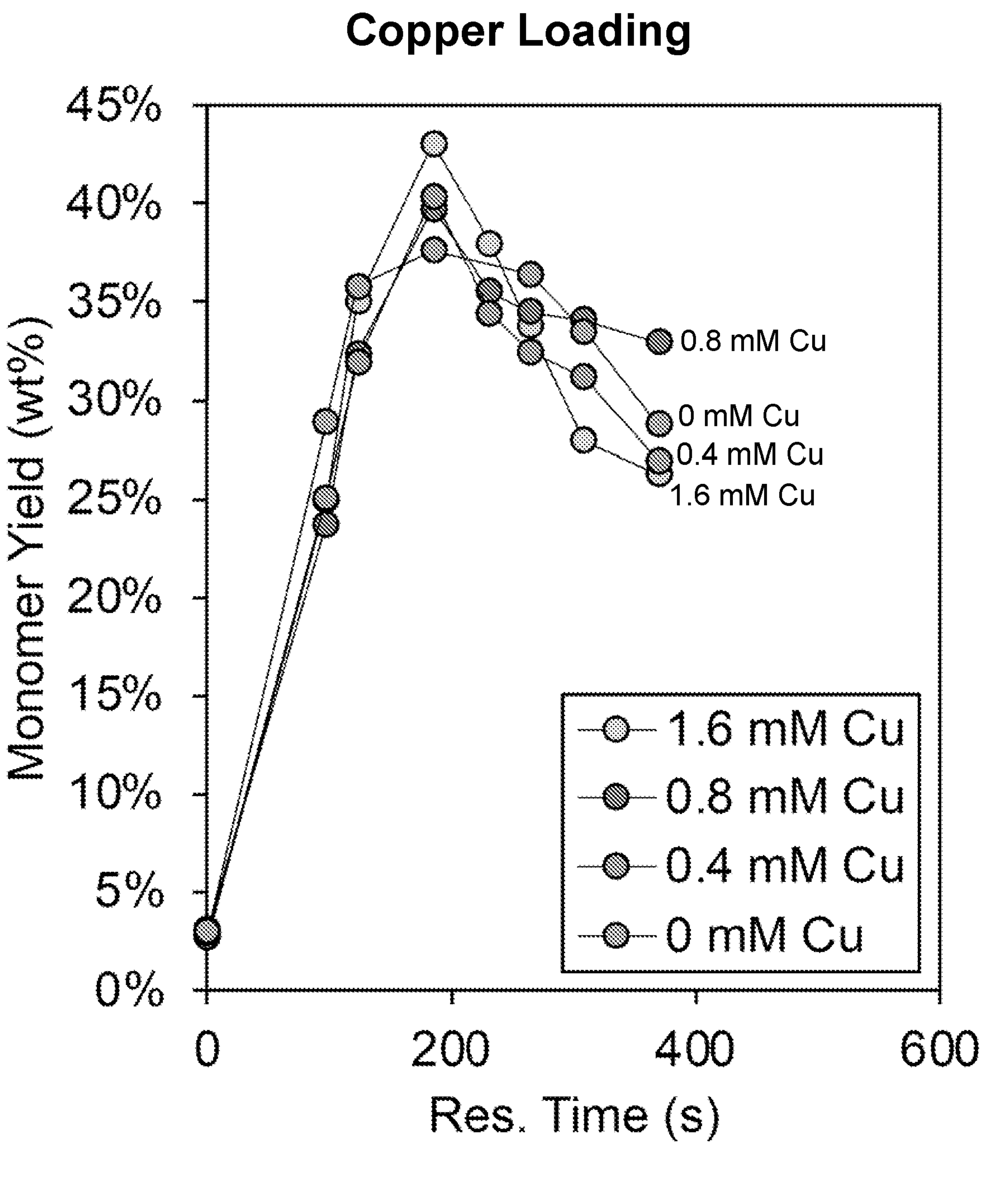

Copper Loading
Monomer Yield (wt%)
45%
40%
35%
30%
25%
20%
15%
10%
5%
0%
0
200
400
600
Res. Time (s)
0.8 mM Cu
0 mM Cu
0.4 mM Cu
1.6 mM Cu
1.6 mM Cu
0.8 mM Cu
0.4 mM Cu
0 mM Cu


FIG. 8

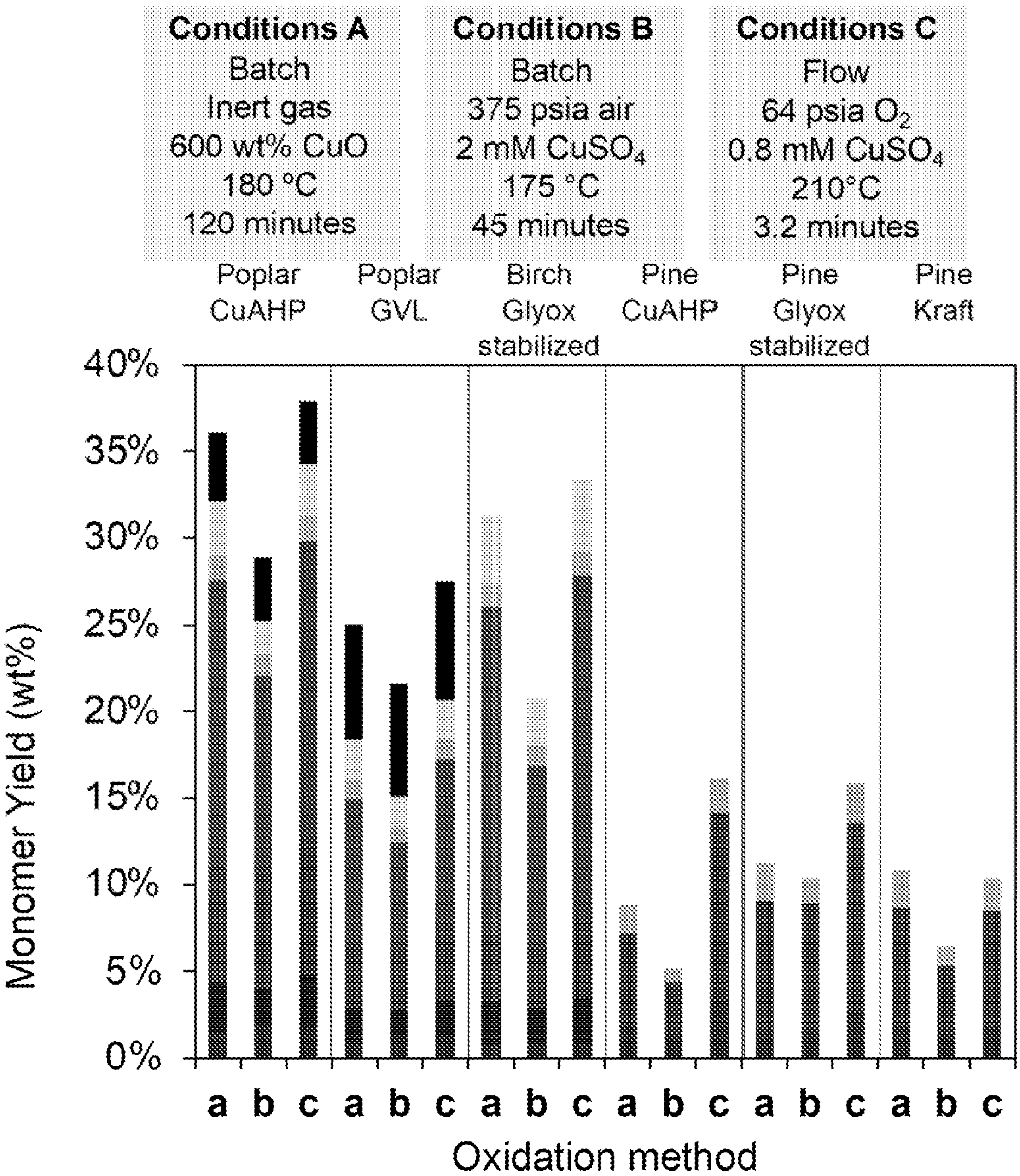
Conditions A
Batch
Inert gas
600 wt% CuO
180 °C
120 minutes
Conditions B
Batch
375 psia air
2 mM $CuSO_4$
175 °C
45 minutes
Conditions C
Flow
64 psia $O_2$
0.8 mM $CuSO_4$
210°C
3.2 minutes
Poplar CuAHP
Poplar GVL
Birch Glyox stabilized
Pine CuAHP
Pine Glyox stabilized
Pine Kraft
40%
35%
30%
25%
20%
15%
10%
5%
0%
Monomer Yield (wt%)
a b c a b c a b c a b c a b c a b c
Oxidation method

FIG. 9A

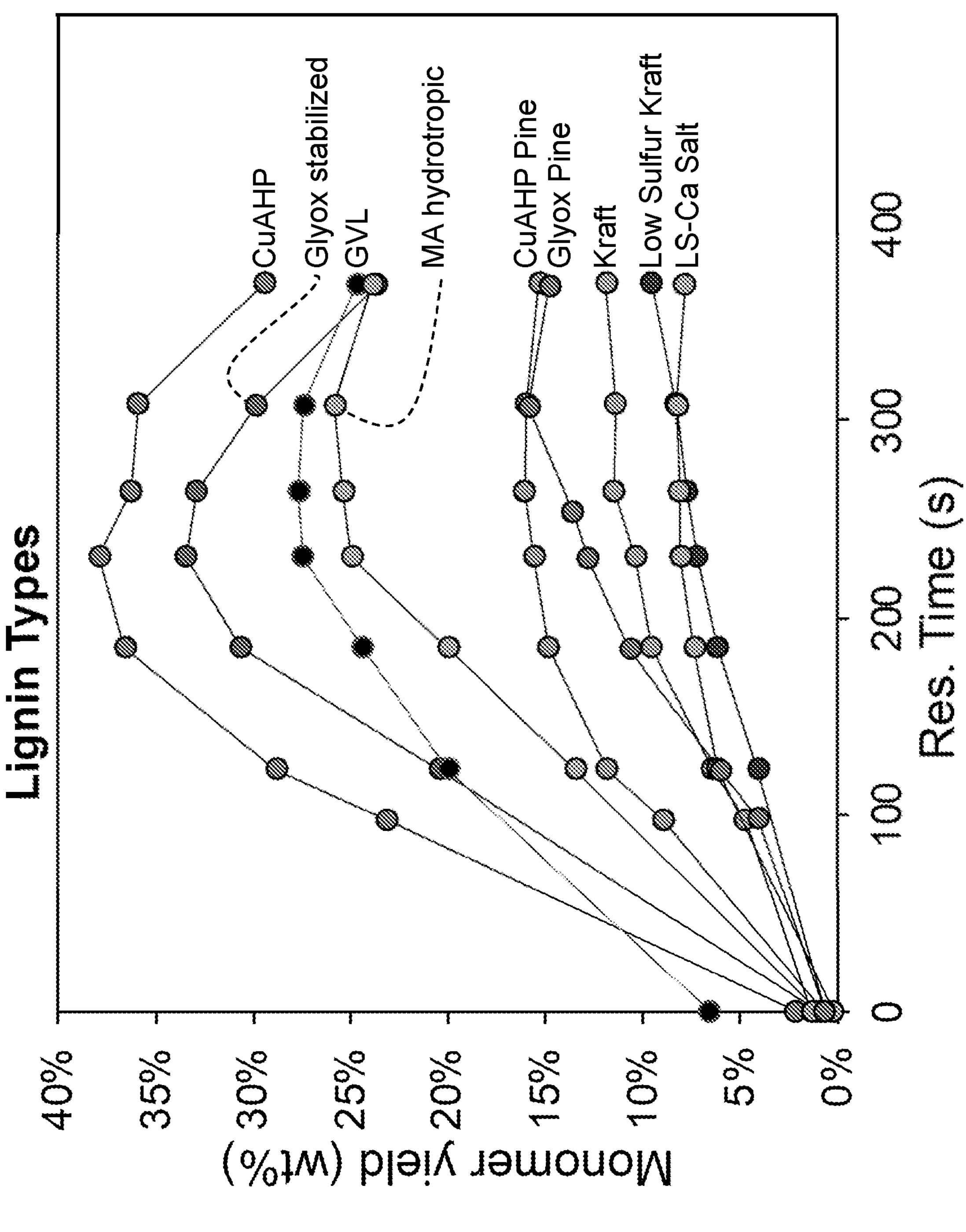
Lignin Types
Monomer yield (wt%)
0%
5%
10%
15%
20%
25%
30%
35%
40%
Res. Time (s)
0
100
200
300
400
CuAHP
Glyox stabilized
GVL
MA hydrotropic
CuAHP Pine
Glyox Pine
Kraft
Low Sulfur Kraft
LS-Ca Salt

FIG. 9B

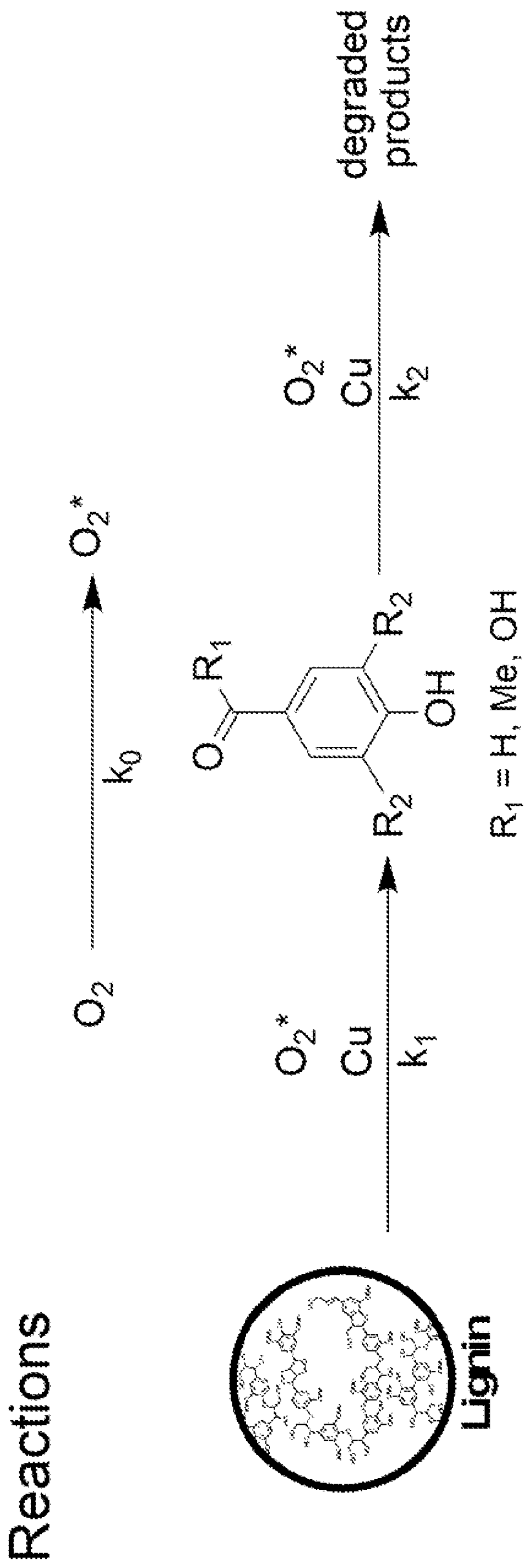
Reactions
Lignin
$O_2^*$
Cu
$k_1$
$O_2$
$k_0$
$O_2^*$
$R_1$ = H, Me, OH
$O_2^*$
Cu
$k_2$
degraded products

FIG. 10A

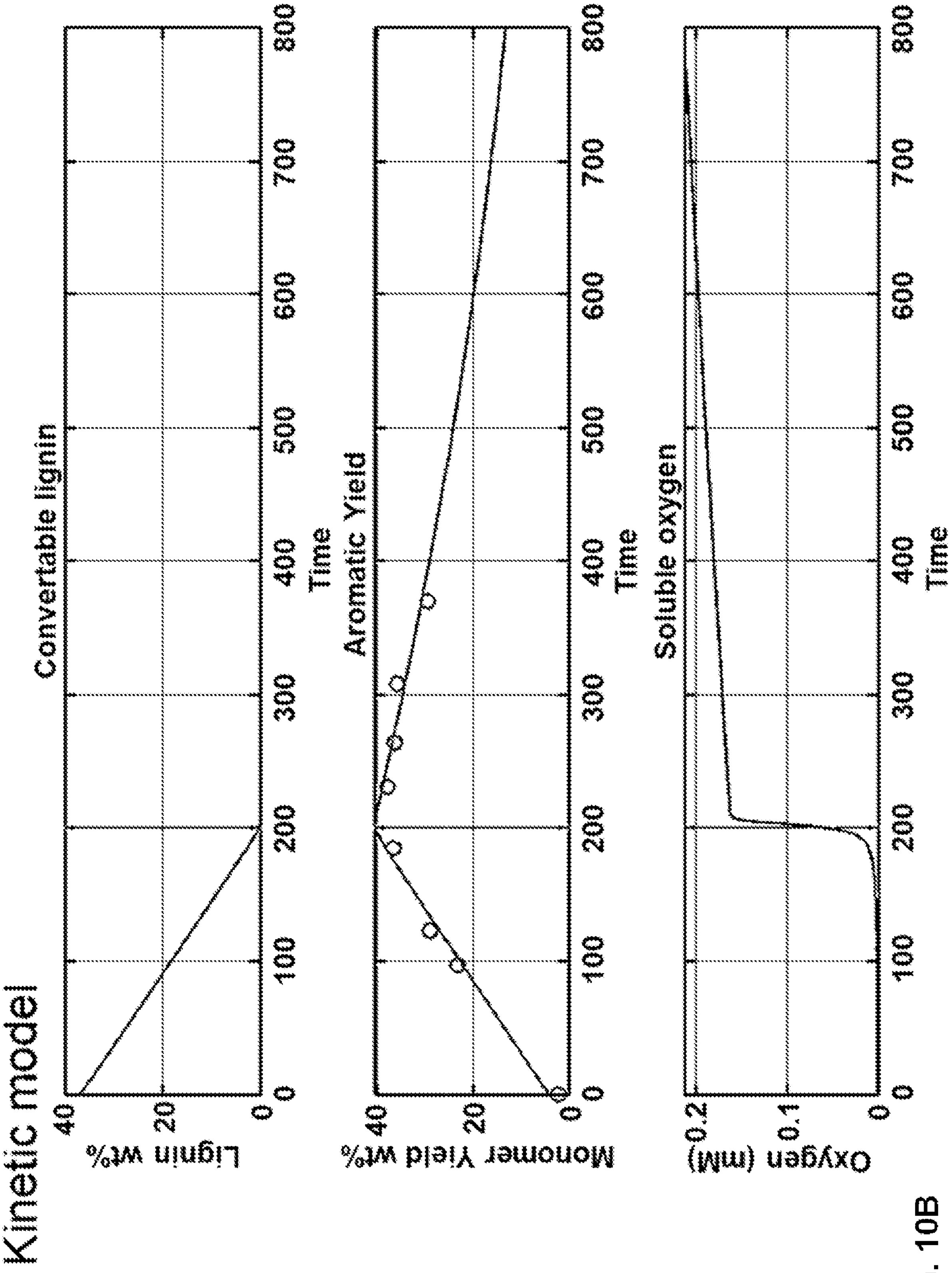
Kinetic model
Convertable lignin
Lignin wt%
Time
Aromatic Yield
Monomer Yield wt%
Time
Soluble oxygen
Oxygen (mM)
Time

FIG. 10B

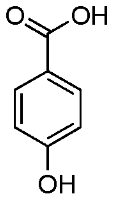

*p*-hydroxybenzoic acid

**FIG. 11A**

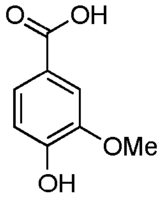

vanillic acid

**FIG. 11B**

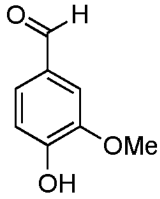

vanillin

**FIG. 11C**

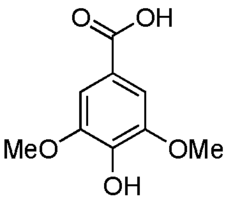

syringic acid

**FIG. 11D**

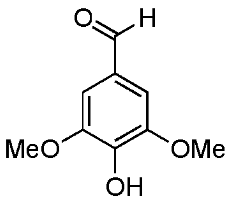

syringaldehyde

**FIG. 11E**

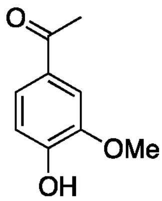

acetovanillone

**FIG. 11F**

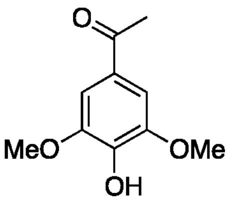

acetosyringone

**FIG. 11G**

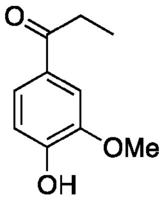

propylvanillone

**FIG. 11H**

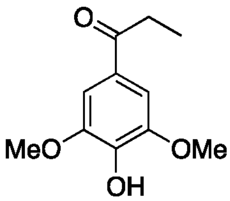

propylsyringone

**FIG. 11I**

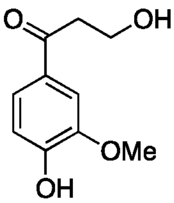

β-hydroxypropiovanillone

**FIG. 11J**

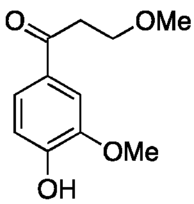

β-methoxypropiovanillone

**FIG. 11K**

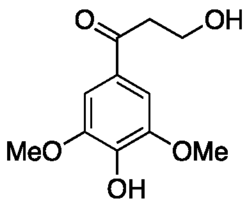

β-hydroxypropiosyringone

**FIG. 11L**

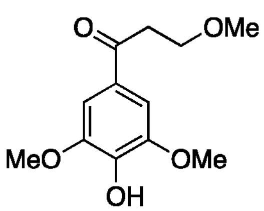

β-methoxypropiosyringone

**FIG. 11M**

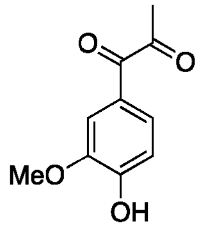

1-(4-hydroxy-3-methoxyphenyl)-1,2-propanedione

**FIG. 11N**

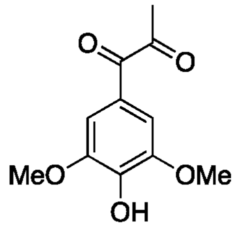

1-(4-hydroxy-3,5-dimethoxyphenyl)-1,2-propanedione

**FIG. 11O**

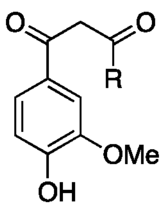

R = H, OH, alkoxy (*e.g.,* C1-C6)

**FIG. 11P**

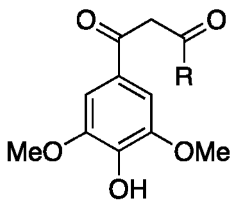

R = H, OH, alkoxy (*e.g.,* C1-C6)

**FIG. 11Q**

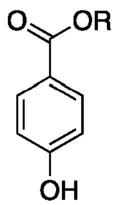

*p*-hydroxybenzoic acid alkyl ester (R = alkyl (*e.g.*, C1-C6))

**FIG. 11R**

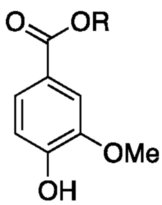

vanillic acid alkyl ester (R = alkyl (*e.g.*, C1-C6))

**FIG. 11S**

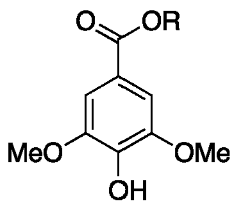

syringic acid alkyl ester (R = alkyl (*e.g.*, C1-C6))

**FIG. 11T**

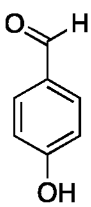

*p*-hydroxybenzaldehyde

**FIG. 11U**

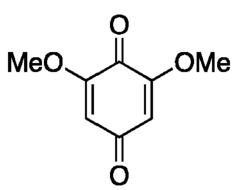

2,6-dimethoxybenzoquinone

**FIG. 11V**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 63287592
- US 20200332376 A1, Hegg
- US 10906856 B2, Bertella
- US 8969534 B2
- WO 2014068590 A
- WO 2020181171 A


### Non-patent literature cited in the description


- **ARAUJO J. D. P.** *Catalysis Today*, 2009, vol. 147, 330-335
- **ALI, MD. E.** ; **RAHMAN, MD. M.** ; **SARKAR, S. M.** ; **HAMID, S. B. A.** Heterogeneous Metal Catalysts for Oxidation Reactions. *Journal of Nanomaterials*, 2014, 1-23
- **ARÁUJO, J. D. P.** ; **GRANDE, C. A.** ; **RODRIGUES, A. E.** Structured Packed Bubble Column Reactor for Continuous Vanillin Production from Kraft Lignin. *Catal. Today*, 2009, vol. 147, S330-S335
- **BASSETT, A. W.** ; **HONNIG, A. E.** ; **BREYTA, C. M.** ; **DUNN, I. C.** ; **LA SCALA, J. J.** ; **STANZIONE, J. F.** III Vanillin-Based Resin for Additive Manufacturing. *ACS Sustainable Chem. Eng.*, 2020, vol. 8, 5626-5635
- **BECKHAM, G. T.** ; **JOHNSON, C. W.** ; **KARP, E. M.** ; **SALVACHIA, D.** ; **VARDON, D. R.** Opportunities and Challenges in Biological Lignin Valorization. *Curr. Opin. Biotechnol.*, 2016, vol. 42, 40-53
- **BERTELLA S** ; **BERNARDES FIGUEIRÊDO M** ; **DE ANGELIS G** ; **MOUREZ M** ; **BOURMAUD C** ; **AMSTAD E** ; **LUTERBACHER JS**. Extraction and Surfactant Properties of Glyoxylic Acid-Functionalized Lignin. *ChemSusChem*, 07 June 2022, vol. 15 (15), e202200270
- **BJØRSVIK, H-R.** ; **MINISCI, F.** Fine Chemicals from Lignosulfonates 1. Synthesis of Vanillin by Oxidation of Lignosulfonates. *Org. Proc. Res. Dev.*, 1999, vol. 3, 330-340
- **BOSQUE, I.** ; **MAGALLANES, G.** ; **RIGOULET, M.** ; **KÄRKÄS, M. D.** ; **STEPHENSON, C. R. J.** Redox Catalysis Facilitates Lignin Depolymerization. *ACS Cent. Sci.*, 2017, vol. 3, 621-628
- Sequential Oxidation - Depolymerization Strategies for Lignin Conversion to Low Molecular Weight Aromatic Chemicals. **CUI, Y.** ; **GOES, S. L.** ; **STAHL, S. S.** Advances in Inorganic Chemistry: Catalysis in Biomass Conversion. Academic Press, 2021, vol. 77, 99-136
- **DAS, A.** ; **RAHIMI, A.** ; **ULBRICH, A.** ; **ALHERECH, M.** ; **MOTAGAMWALA, A. H.** ; **BHALLA, A.** ; **SOUSA, L. C.** ; **BALAN, V.** ; **DUMESIC, J. A.** ; **HEGG, E. L.** Lignin Conversion to Low-Molecular-Weight Aromatics via an Aerobic Oxidation-Hydrolysis Sequence: Comparison of Different Lignin Sources. *ACS Sustainable Chem. Eng.*, 2018, vol. 6 (3), 367-3374
- **DU, X.** ; **TRICKER, A. W.** ; **YANG, W.** ; **KATAHIRA, R.** ; **LIU, W.** ; **KWOK, T. T.** ; **GOGOI, P.** ; **DENG, Y.** Oxidative Catalytic Fractionation and Depolymerization of Lignin in a One-Pot Single-Catalyst System. *ACS Sustainable Chem. Eng.*, 2021, vol. 9, 7719-7727
- **FANG, W.** ; **SIXTA, H.** Advanced biorefinery based on the fractionation of biomass in γ-valerolactone and water. *ChemSusChem*, 2015, vol. 8 (1), 73-76
- **FARGUES, C.** ; **MATHIAS, A.** ; **SILVA, J.** ; **RODRIGUES, A.** Kinetics of Vanillin Oxidation. *Chem. Eng. Technol.*, 1996, vol. 19, 127-136
- **GELOSIA, M.** ; **INGLES, D.** ; **POMPILI, E.** ; **D'ANTONIO, S.** ; **CAVALAGLIO, G.** ; **PETROZZI, A.** ; **COCCIA, V.** Fractionation of lignocellulosic residues coupling steam explosion and organosolv treatments using green solvent γ-valerolactone. *Energies*, 2017, vol. 10 (9), 1264
- **GEWIRTH, A. A.** ; **VARNELL, J. A.** ; **DIASCRO, A. M.** Nonprecious Metal Catalysts for Oxygen Reduction in Heterogeneous Aqueous Systems. *Chemical Reviews*, 2018, vol. 118, 2313-2339
- **GIACOBBE FW**. Oxygen Permeability of Teflon-PFA Tubing. *Journal of Applied Polymer Science*, 1990, vol. 39 (5), 1121-1132
- **GREENE, J. F.** ; **PREGER, Y.** ; **ROOT, T. W.** ; **STAHL, S. S.** *Org. Process Res. Dev.*, 2015, vol. 19 (7), 858, 864
- **GUTMANN, B.** ; **CANTILLO, D.** ; **KAPPE, C. O.** Continuous Flow Technology - A Tool for the Safe Manufacturing of Active Pharmaceutical ingredients. *Angew. Chem. Int. Ed.*, 2015, vol. 54, 6688-6728

- **HOCKING, M. B.** Vanillin: Synthetic Flavoring from Spent Sulfite Liquor. *J. Chem. Educ.*, 1997, vol. 74, 1055-1059
- **KAUR, N.** ; **KISHORE, D.** *Catal Surv Asia*, 2013, vol. 17 (1), 20-42
- **KUMAR AK** ; **SHARMA S**. Recent Updates on Different Methods of Pretreatment of Lignocellulosic Feedstocks: A Review. *Bioresour. Bioprocess*, 2017, vol. 4, 7
- **KUMAR, P.** ; **BARRETT, D. M.** ; **DELWICHE, M. J.** ; **STROEVE, P.** Methods for Pretreatment of Lignocellulosic Biomass for Efficient Hydrolysis and Biofuel Production. *Industrial & Engineering Chemistry Research*, 2009, vol. 48 (8), 3713-3729
- **LANCEFIELD, C. S.** ; **OJO, O. S.** ; **TRAN, F.** ; **WESTWOOD, N. J.** Isolation of Functionalized Phenolic Monomers through Selective Oxidation and C-O Bond Cleavage of the β-O-4 Linkages in Lignin. *Angew. Chem., Int. Ed.*, 2015, vol. 54, 258-262
- Billion-Ton Report: Advancing Domestic Resources for a Thriving Bioeconomy. **LANGHOLTZ, M. H.** ; **STOKES, B. J.** ; **EATON, L. M.** Economic Availability of Feedstocks; Technical report. U.S. Department of Energy, July 2016, vol. 1
- **LE, H. Q.** ; **MA, Y.** ; **BORREGA, M.** ; **SIXTA, H.** Wood biorefinery based on γ-valerolactone/water fractionation. *Green Chem*, 2016, vol. 18 (20), 5466-5476
- **LE, H. Q.** ; **ZAITSEVA, A.** ; **POKKI, J. P** ; **STAHL, M.** ; **ALOPAEUS, V.** ; **SIXTA, H.** Solubility of organosolv lignin in γ-valerolactone/water binary mixtures. *ChemSusChem*, 2016, vol. 9 (20), 2939-2947
- **LLEVOT, A.** ; **GRAU, E.** ; **CARLOTTI, S.** ; **GRELIER, S.** ; **CRAMAIL, H.** From Lignin-derived Aromatic Compounds to Novel Biobased Polymers. *Macromol. Rapid Commun.*, 2016, vol. 37, 9-28
- **LUO, H.** ; **WEEDA, E. P.** ; **ALHERECH, M.** ; **ANSON, C. W.** ; **KARLEN, S. D.** ; **CUI, Y.** ; **FOSTER, C. E.** ; **STAHL, S. S.** *J. Am. Chem. Soc.*, 2021, vol. 143 (37), 15462-15470
- **LUTERBACHER, J. S.** ; **RAND, J. M.** ; **ALONSO, D. M.** ; **HAN, J.** ; **YOUNGQUIST, J. T.** ; **MARAVELIAS, C. T.** ; **PFLEGER, B. F.** ; **DUMESIC, J. A.** Nonenzymatic sugar production from biomass using biomass-derived γ-valerolactone. *Science*, 2014, vol. 343 (6168), 277-280
- **LUTERBACHER, J. S.** ; **ALONSO, D. M.** ; **RAND, J. M.** ; **QUESTELL-SANTIAGO, Y. M.** ; **YEAP, J. H.** ; **PFLEGER, B. F.** ; **DUMESIC, J. A.** Solvent-enabled nonenyzmatic sugar production from biomass for chemical and biological upgrading. *ChemSusChem*, 2015, vol. 8 (8), 1317-1322
- **PEREZ, J. M.** ; **KONTUR, W. S.** ; **ALHERECH, M.** ; **COPLIEN, J.** ; **KARLEN, S. D.** ; **STAHL, S. S.** ; **DONOHUE, T. J.** ; **NOGUERA, D. R.** Funneling Aromatic Products of Chemically Depolymerized Lignin into 2-Pyrone-4-6-Dicarboxylic Acid with Novosphingobium Aromaticivorans. *Green Chem*, 2019, vol. 21, 1340-1350
- **PETERSEN, T. P.** ; **POLYZOS, A.** ; **O'BRIEN, M** ; **ULVEN, T.** ; **BAXENDALE, I. R.** ; **LEY, S. V.** The Oxygen-Mediated Synthesis of 1,3-Butadiynes in Continuous Flow: Using Teflon AF-2400 to Effect Gas/Liquid Contact. *ChemSusChem*, 2012, vol. 5, 274-277
- **PINNAU I** ; **TOY LG**. Gas and Vapor Transport Properties of Amorphous Perfluorinated Copolymer Memberanes Based on 2,2-Bistrifluoromethyl-4,5-difluoro-1,3-dioxole/tetrafluoroethylene. *Journal of Membrane Science*, 1996, vol. 109, 125-133
- **RAFIEE, M.** ; **ALHERECH, M.** ; **KARLEN, S. D.** ; **STAHL, S. S.** Electrochemical Aminoxyl-Mediated Oxidation of Primary Alcohols in Lignin to Carboxylic Acids: Polymer Modifications and Depolymerization. *J. Am. Chem. Soc.*, 2019, vol. 141, 15266-15276
- **ROBERTS, V. M.** ; **STEIN, V.** ; **REINER, T.** ; **LEMONIDOU, A.** ; **LI, X.** ; **LERCHER, J. A.** Towards Quantitative Catalytic Lignin De-polymerization. *Chem. Eur. J.*, 2011, vol. 17, 5939-5948
- **SCHUTYSER, W.** ; **KRUGER, J.S.** ; **ROBINSON, A.M.** ; **KATAHIRA, R.** ; **BRANDNER, D.G.** ; **CLEVELAND, N.S.** ; **MITTAL, A.** ; **PETERSON, D.J.** ; **MEILAN, R.** ; **ROMÁN-LESHKOV, Y.** Revisiting alkaline aerobic lignin oxidation. *Green Chemistry*, 2018, vol. 20, 3828-3844
- **SRIHAR, P.** ; **ARAUJO, J. D.** ; **RODRIGUES, A.** Modeling of Vanillin Production in a Structured Bubble Column Reactor. *Catal. Today*, 2005, vol. 105, 574-581
- **SUN, T.** ; **TIAN, B.** ; **SU, C.** Recent advances in Fe (or Co)/N/C electrocatalysts for the oxygen reduction reaction in polymer electrolyte membrane fuel cells. *J. Mater. Chem. A.*, 2017, vol. 5, 18933-18950
- **SUN, Z.** ; **FRIDRICH, B.** ; **DE SANTI, A.** ; **ELANGOVAN, S.** ; **BARTA, K.** Bright Side of Lignin Depolymerization: Toward New Platform Chemicals. *Chem. Rev.*, 2018, vol. 118, 614-678
- **TUCK, C. O.** ; **PÉREZ, E.** ; **HORVÁTH, I. T.** ; **SHELDON, R. A.** ; **POLIAKOFF, M.** Valorization of Biomass: Deriving More Value from Waste. *Science*, 2012, vol. 337, 695-699
- **WU, M.** ; **LIU, J.-K.** ; **YAN, Z.-Y.** ; **WANG, B.** ; **ZHANG, X.-M.** ; **XU, F.** ; **SUN, R.-C.** Efficient recovery and structural characterization of lignin from cotton stalk based on a biorefinery process using a γ-valerolactone/water system. *RSC Adv*, 2016, vol. 6 (8), 6196-6204

- **XANTHOPOULOU, E.** ; **TERZOPOULOU, Z.** ; **ZAMBOULIS, A.** ; **PAPADOPOULOS, L.** ; **TSONGAS, K.** ; **TZETZIS, D.** ; **PAPAGEORGIOU, G. Z.** ; **BIKIARIS, D. N.** Poly(Propylene Vanillate): A Sustainable Lignin-Based Semicrystalline Engineering Polyester. *ACS Sustainable Chem. Eng.*, 2021, vol. 9, 1383-1397